# FASCICULE DE BREVET EUROPEEN

(11) **EP 1 642 567 B1**
(45) Date de publication et mention de la délivrance du brevet: **03.11.2010**
(21) Numéro de dépôt: 05291981.8
(22) Date de dépôt: 23.09.2005
(51) Int. Cl.: A61K 8/49, A61Q 5/10

(54) **Composition tinctoriale comprenant un colorant direct naphthoylène-benzimidazolium, procédé de mise en oeuvre et utilisations**
Färbemittel enthaltend eine direktziehende Naphthoylen-benzimidazoliumverbindung, Herstellungsverfahren und Verwendungen
Colouring composition comprising naphthoylene-benzimidazolium compound as direct dye, preparation and uses

(30) Priorité: 04.10.2004 FR 0410453
(43) Date de publication de la demande: 05.04.2006
(73) Titulaire: L'Oréal, 75008 Paris (FR)
(72) Inventeur: Lagrange, Alain, 77700 Coupvray (FR)
(74) Mandataire: Casalonga, Axel

(56) Documents cités:
- DE-A1- 2 519 169
- US-A- 3 459 489

## Description

La présente demande a pour objet une composition tinctoriale pour la teinture des fibres kératiniques comprenant dans un milieu de teinture approprié au moins un colorant direct particulier de type naphthoylène-benzimidazolium.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont formés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Toutefois, les associations base-coupleur de l'état de la technique ne donnent pas encore toute satisfaction en terme de luminosité des nuances (chromaticité), de ténacité ou de sélectivité.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser pauser pour permettre aux molécules colorées de pénétrer, par diffusion, à l'intérieur du cheveu, puis à rincer les fibres.

Il est connu par exemple d'utiliser des colorants directs nitrés benzèniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des interactions entre les colorants directs et la fibre kératinique. Ces interactions sont faibles et font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre souvent sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et/ou sur la fibre kératinique.

De manière surprenante et avantageuse, la Demanderesse vient de découvrir de nouvelles compositions pour la teinture des fibres kératiniques, en particulier des fibres kératiniques humaines telles que les cheveux, à base de colorants directs de type naphthoylène-benzimidazolium qui permettent de colorer les cheveux même non sensibilisés de manière intense.

Outre leur avantage en terme d'innocuité, les compositions selon la présente demande permettent l'obtention de nuances très puissantes, peu sélectives et tenaces. Les couleurs obtenues sont jaune brillant, orange, rouge, ou violet à violet-bleu. Certaines compositions permettent même l'obtention de couleurs fluorescentes.

En outre, ces compositions présentent une bonne innocuité.

Un premier objet de la présente demande consiste en l'utilisation d'une composition tinctoriale pour la teinture des fibres kératiniques, en particulier des fibres kératiniques humaines telles que les cheveux, comprenant au moins un colorant direct naphthoylène-benzimidazolium particulier de formule générale (I) ou des formules mésomères correspondantes.

Une variante de ce premier objet consiste en une composition tinctoriale comprenant au moins un colorant direct naphthoylène-benzimidazolium de formule (I) et au moins un précurseur de colorant d'oxydation et/ou au moins un agent de conditionnement et/ou au moins un polymère épaississant et/ou au moins un tensioactif.

Un deuxième objet de la présente demande consiste en un procédé de teinture des fibres kératiniques, en particulier des fibres kératiniques humaines telles que les cheveux mettant en oeuvre cette composition.

Un troisième objet de la présente demande consiste en l'utilisation de la composition de la présente invention pour l'obtention sur les fibres kératiniques, en particulier les fibres kératiniques humaines telles que les cheveux de reflets très puissants, peu sélectifs et tenaces.

De préférence, la composition tinctoriale est aqueuse. On entend par « aqueuse » une composition contenant au moins 1% en poids d'eau et de préférence au moins 10% d'eau et encore plus préférentiellement au moins 20% d'eau.

Les colorants directs de type naphthoylène-benzimidazolium utilisables dans les compositions selon la présente invention, correspondent à la formule (I) suivante : 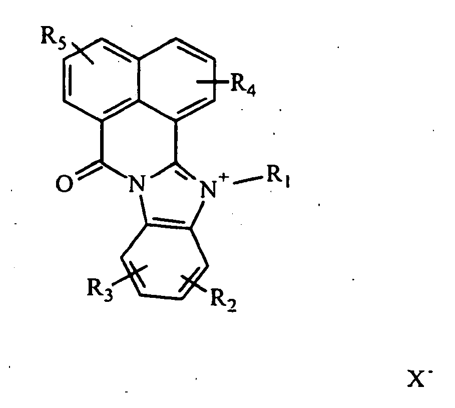 dans laquelle :
R₁ représente un groupement alkyle en C₁-C₁₀ linéaire ou ramifié, aryl alkyle en C₁-C₁₀,
R₂ et R₃ représentent, indépendamment l'un de l'autre, un atome d'hydrogène ; un groupement alkyle en C₁-C₁₀ linéaire ou ramifié, un groupement alkoxy en C₁-C₁₀ linéaire ou ramifié, un atome d'halogène (F, Cl, Br, I), un groupement alcoxy en C₁-C₁₀ carbonyle ; un groupement CF₃,
R₄ et R₅ représentent, indépendamment l'un de l'autre, un atome d'hydrogène ; un atome d'halogène(F, Cl, Br, I), un groupement alkyle en C₁-C₁₀ linéaire ou ramifié ; un groupement alkoxy en C₁-C₁₀ linéaire ou ramifié ; un groupement hydroxy, un groupement amino ; un groupement alkylamino en C₁-C₁₀ linéaire ou ramifié , un groupement dialkylamino en C₁-C₁₀ linéaire ou ramifié , un groupement phényl amino, un groupement diphényl amino, un groupement alkyle en C₁-C₄ amino phényl amino, un groupement amino phényl amino phényl ; un groupement un groupement alkylthio en C₁-C₁₀ linéaire ou ramifié , un groupement phénylthio, un groupement alkyl en C₁-C₁₀ phénylthio, un groupement alkoxy en C₁₋C₁₀ phénylthio, un groupement phényl alkyl en C₁-C₄ thio, morpholino ou R₄ et R₅ forment ensemble un noyau aromatique ou hétérocyclique à 5 à 12 chaînons.

Ces groupements peuvent être éventuellement substitués. Par « substitué » on entend substitué par un ou plusieurs groupements hydroxy, halogène (F, C1, Br, I), alcoxy en C₁-C₁₀, amino, dialkyl en C₁-C₁₀ amino, mono ou dihydroxyalkyl en C₁-C₁₀ amino, amido alcoxy en C₁-C₁₀ carbonyle, acyle en C₂-C₁₀ amino, aryle, cyano.

A moins qu'il n'en soit expressément décrit autrement, les radicaux alkyles ou les parties alkyles des groupes contenant des radicaux alkyles sont en C₁-C₁₀ et peuvent être linéaires ou ramifiés par exemple ce sont des radicaux méthyle, éthyle, n-propyle, iso-propyle, butyle, etc. Un radical alcoxy est un radical alk-O, le radical alkyle ayant la définition donnée ci dessus.

X- représente un contre ion d'un acide minéral ou organique.

De préférence, au moins un des substituants R₂, R₃, R₄, R₅ désigne un atome d'hydrogène.

De préférence, les colorants directs de formule (I) utilisables dans les compositions selon la présente invention sont tels que R₁ représente l'un des groupes suivants : alkyle en C₁-C₅ ; alkyle en C₁-C₅ substitué par un radical OH ; -CH₂-C₆H₄-CN ; -CH₂-C₆H₅.

De préférence, les colorants directs de formule (I) utilisables dans les compositions selon la présente invention sont tels que R₂ et R₃ représentent, indépendamment l'un de l'autre, un atome d'hydrogène ; Br ; Cl ; CF₃, un groupe alkyle en C₁-C₄ ; alcoxy en C₁-C₄ ; alcoxy en C₁-C₆carbonyle, hydroxy alkyle en C₁-C₈.

De manière préférée, les colorants directs de formule (I) utilisables dans les compositions selon la présente invention sont tels que R₄ et R₅ représentent indépendamment l'un de l'autre, un atome d'hydrogène, un groupement alkyle en C₁-C₄ linéaire ou ramifié ; un groupement alkyle en C₁-C₄ amino phényl amino, un groupement amino phényl amino phényl ; un groupement phénylthio, un groupement alkyl en C₁-C₁₀ phénylthio, un groupement alkoxy en C₁₋C₁₀ phénylthio, un groupement phényl alkyl en C₁-C₄ thio, morpholino.

De manière particulière, les colorants directs de formule (I) utilisables dans les compositions selon la présente invention sont choisis parmi les composés suivants, pour lesquels X⁻ représente un contre ion d'un acide minéral ou organique de préférence choisi parmi Cl⁻, I⁻ ,CH₃-C₆H₄-SO₃⁻, CH₃-O- SO₃⁻,

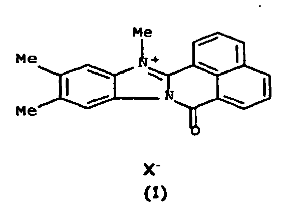

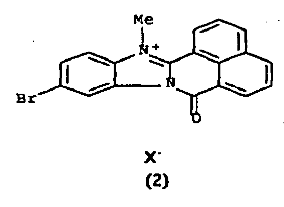

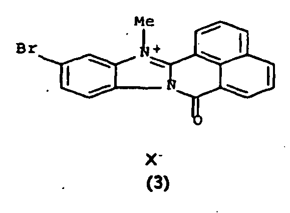

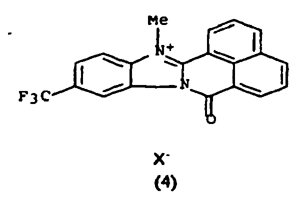

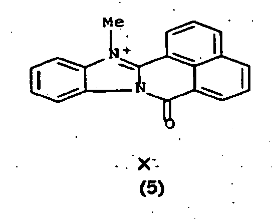

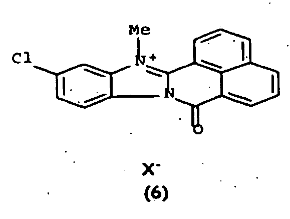

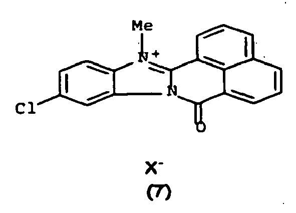

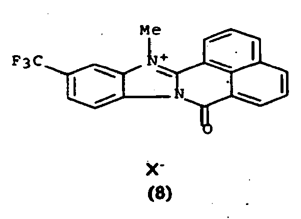

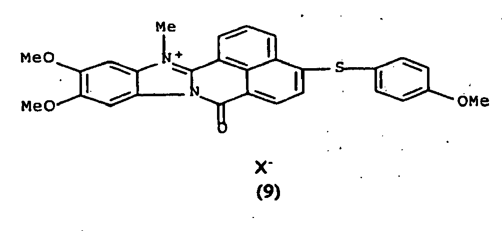

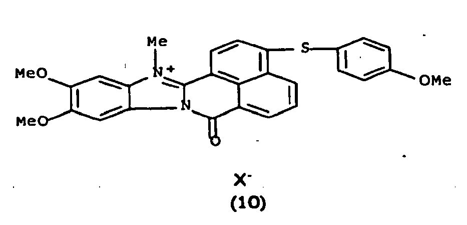

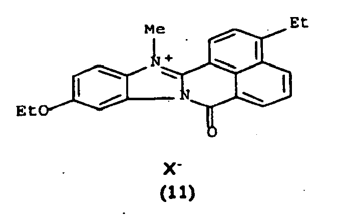

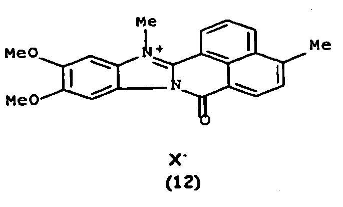

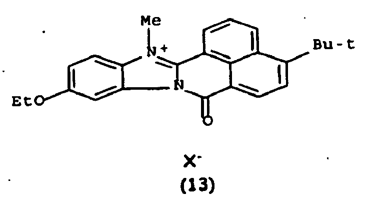

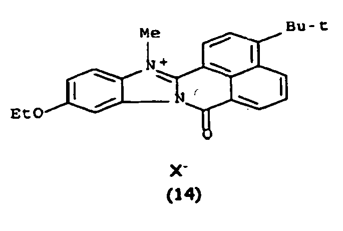

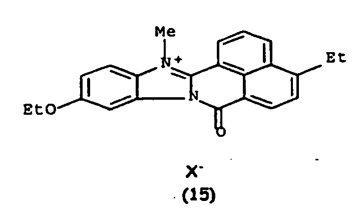

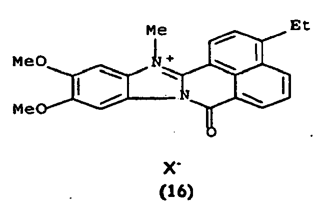

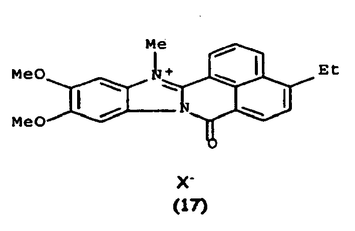

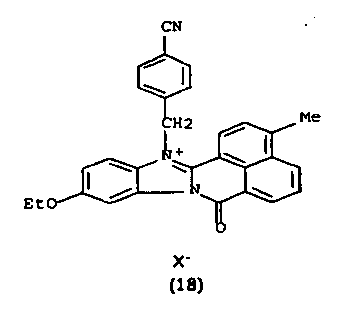

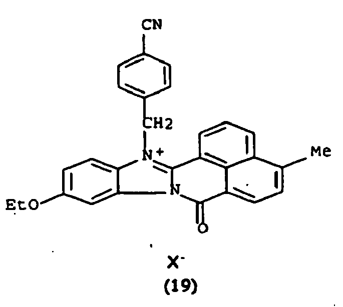

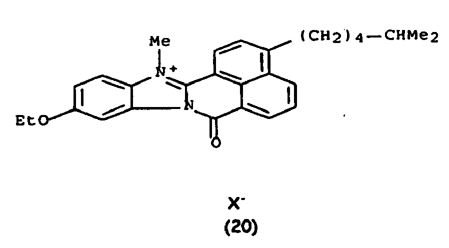

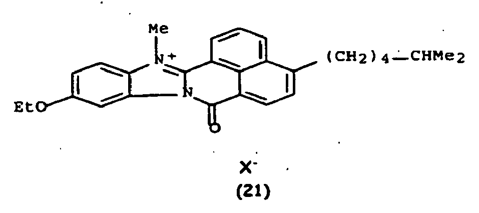

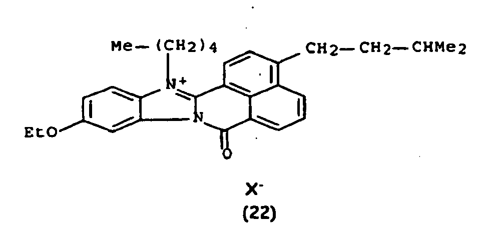

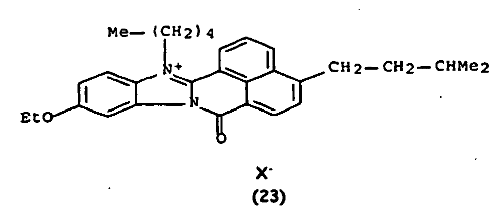

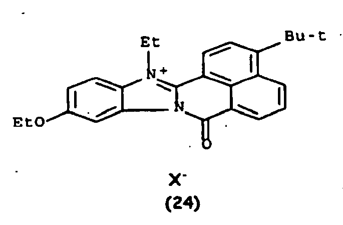

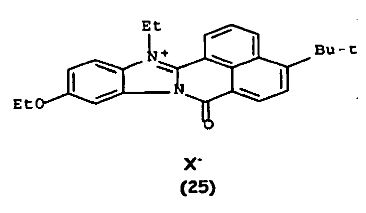

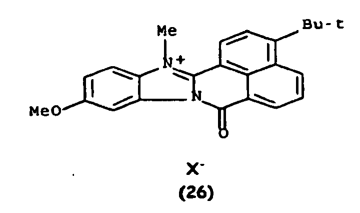

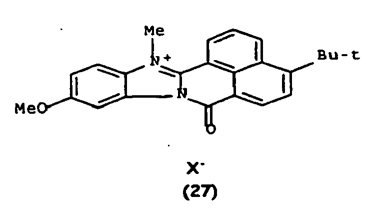

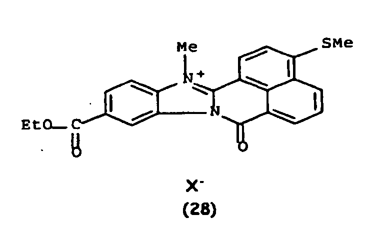

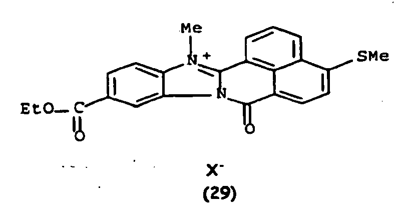

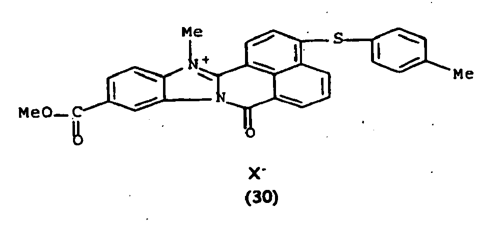

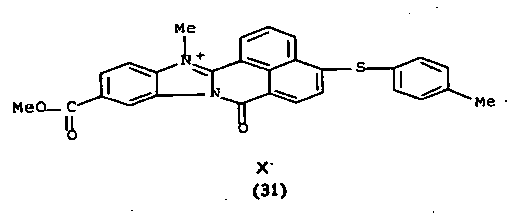

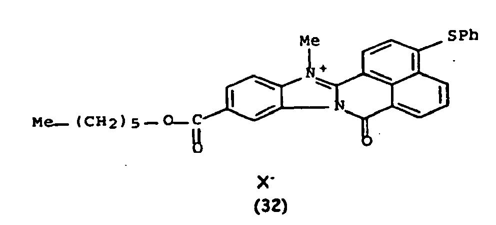

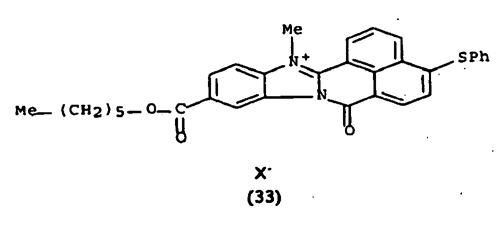

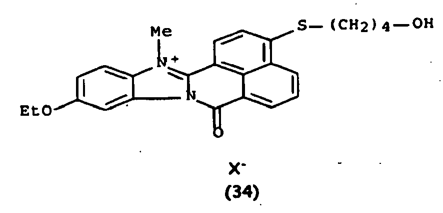

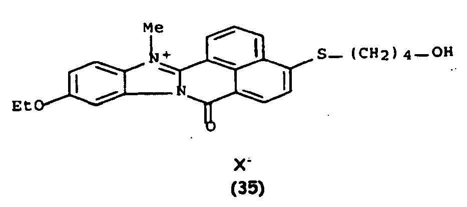

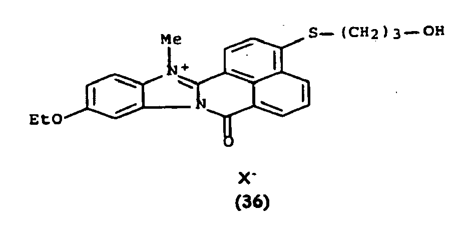

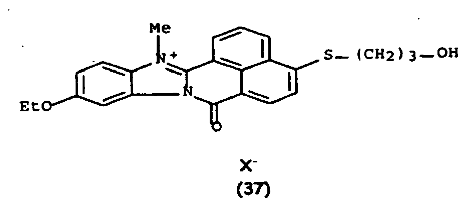

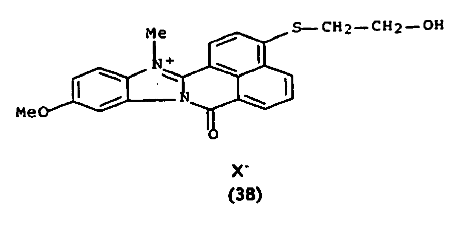

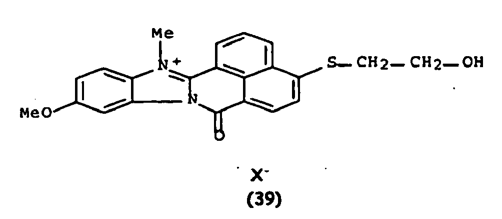

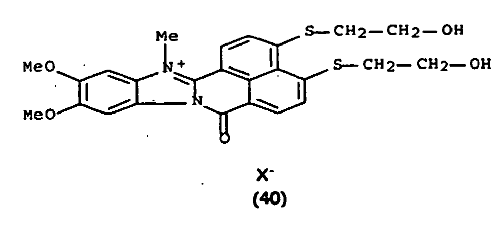

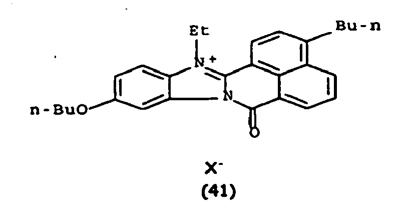

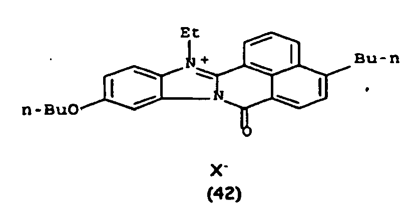

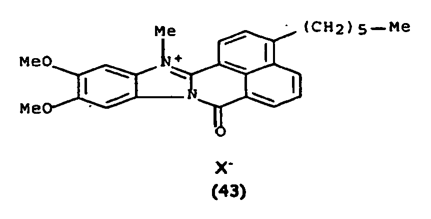

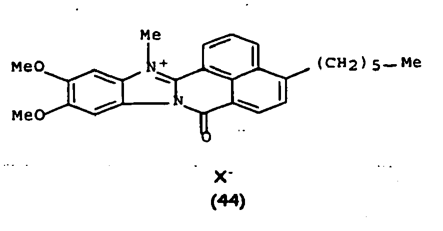

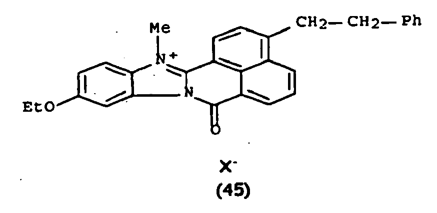

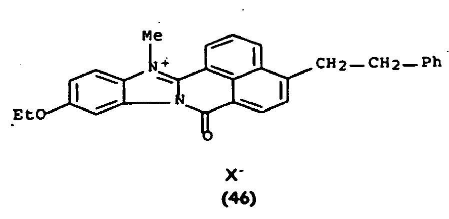

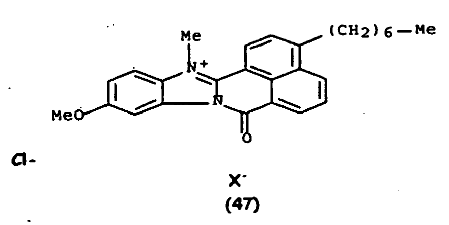

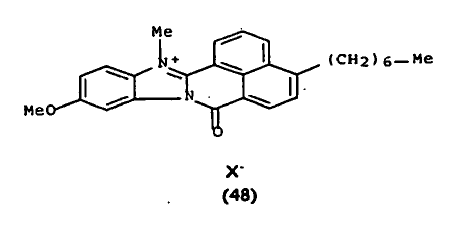

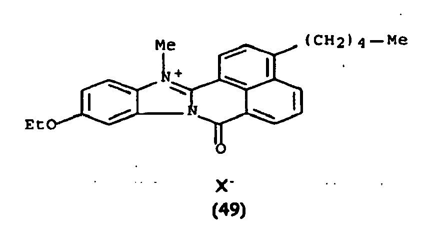

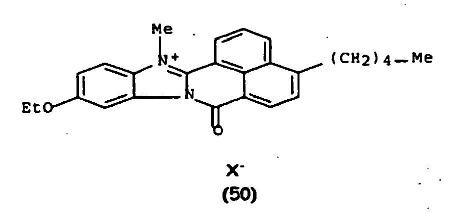

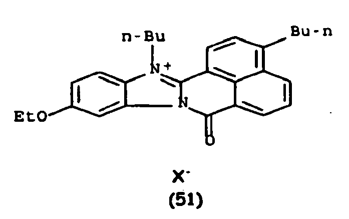

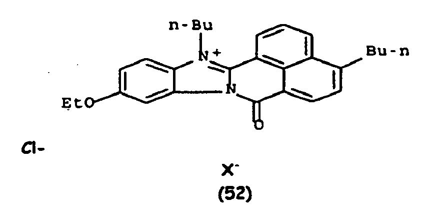

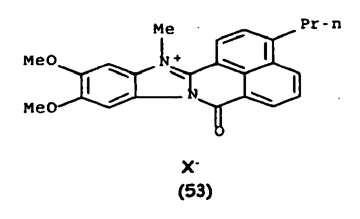

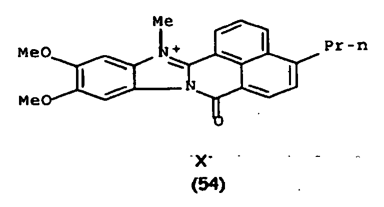

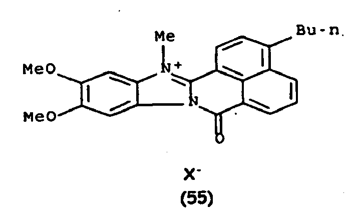

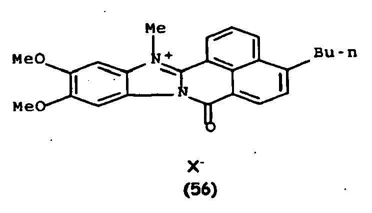

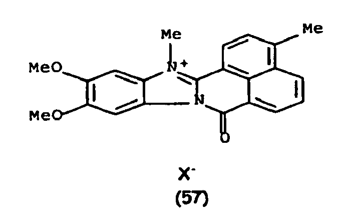

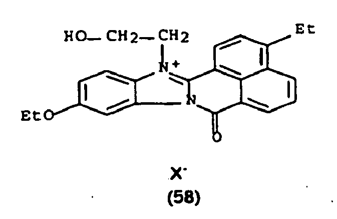

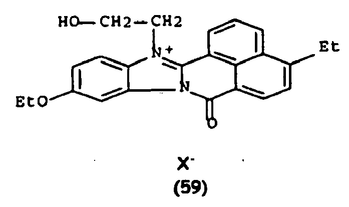

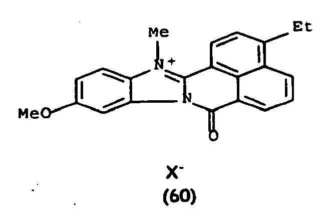

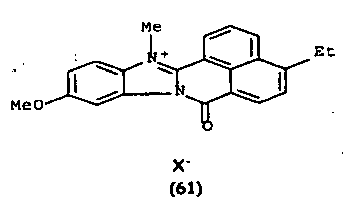

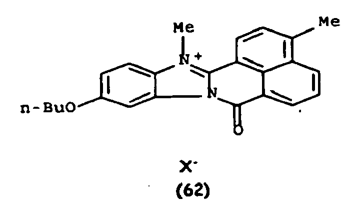

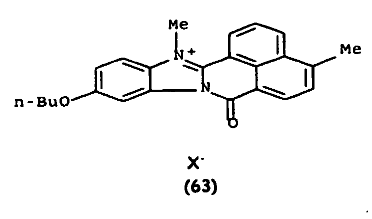

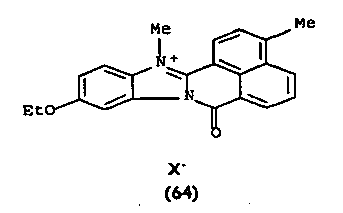

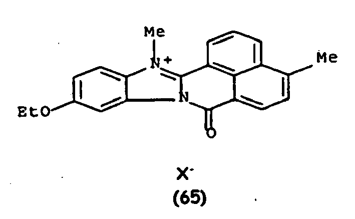

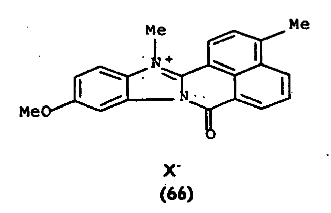

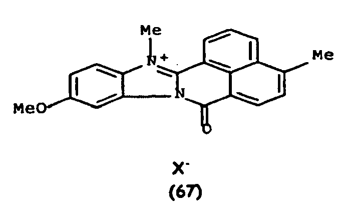

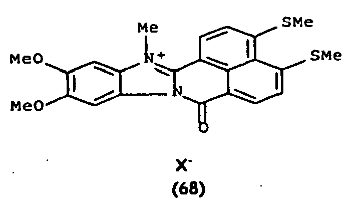

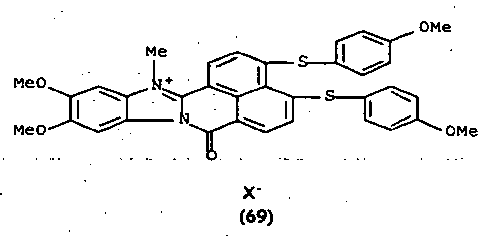

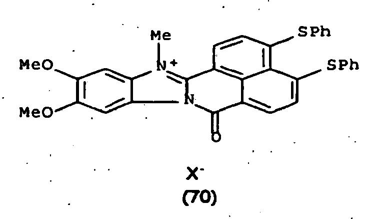

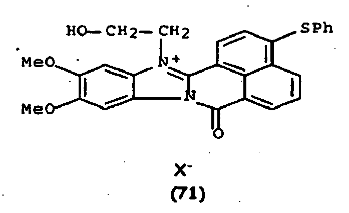

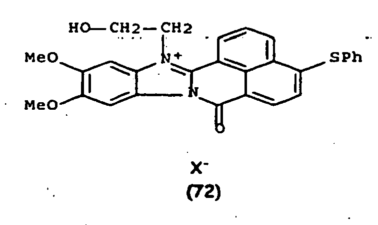

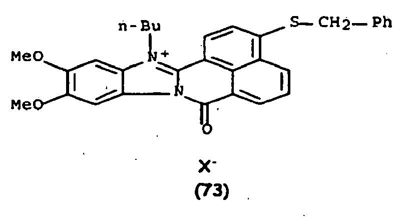

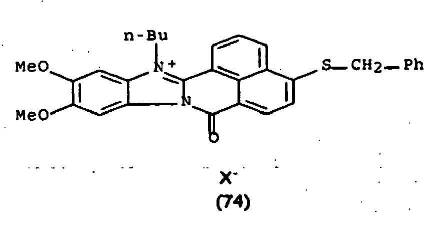

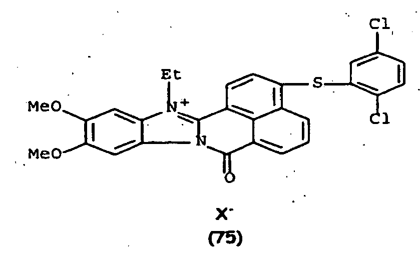

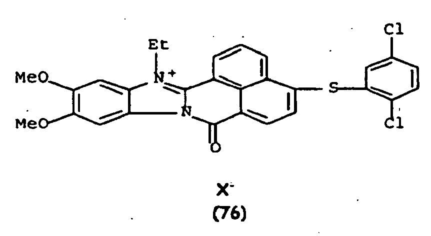

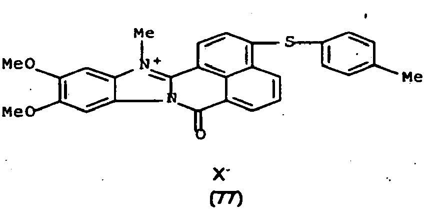

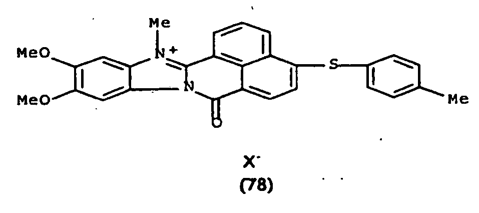

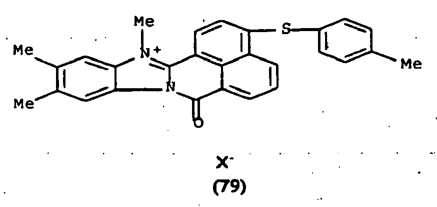

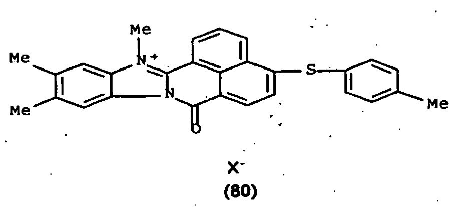

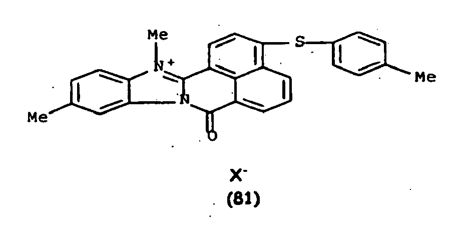

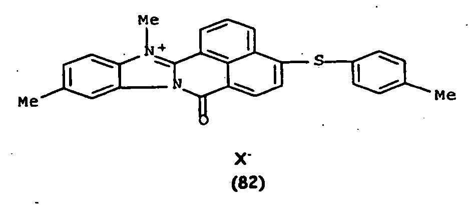

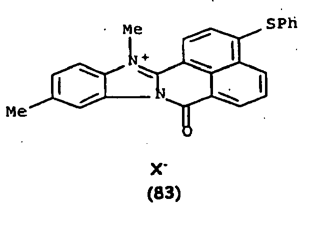

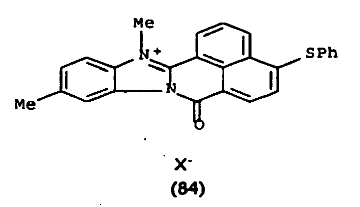

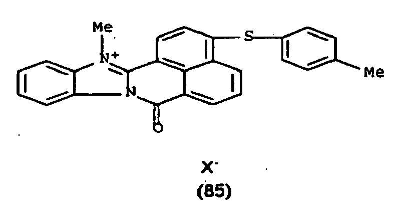

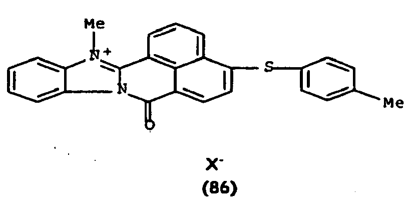

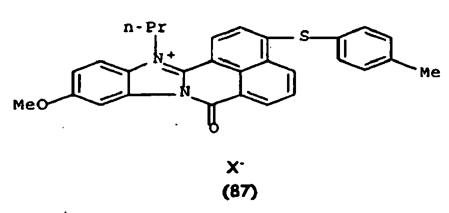

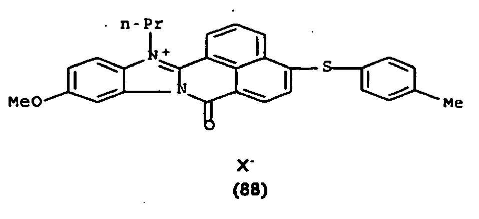

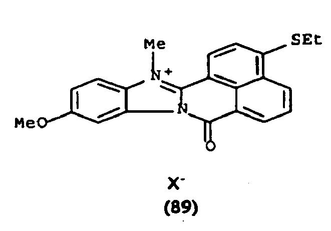

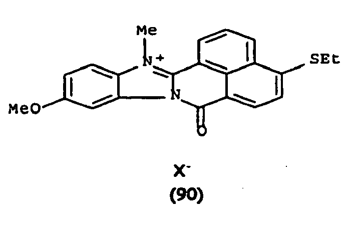

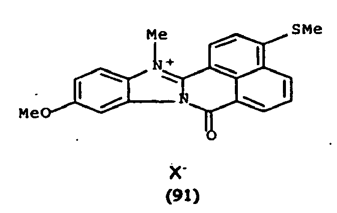

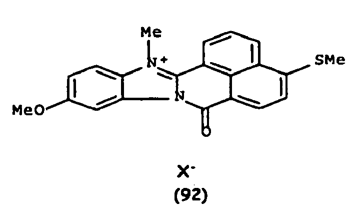

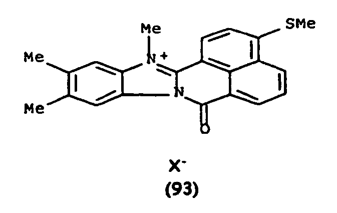

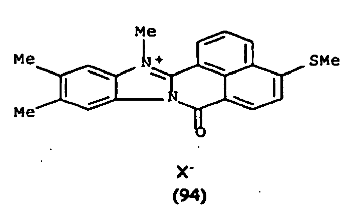

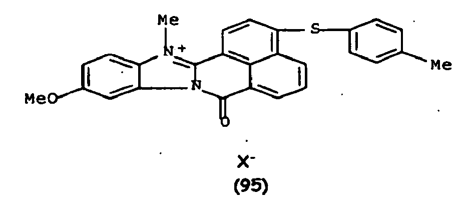

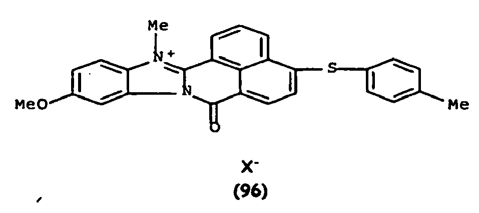

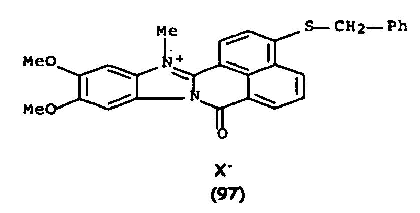

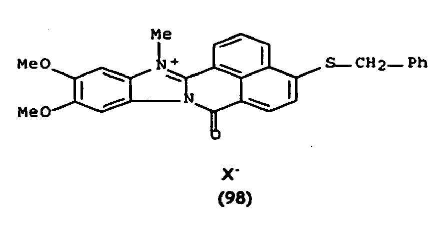

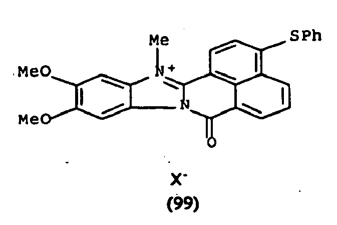

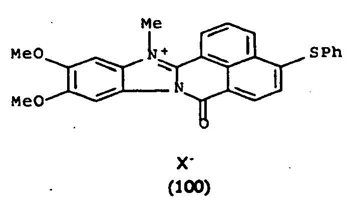

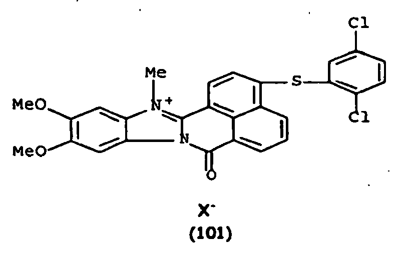

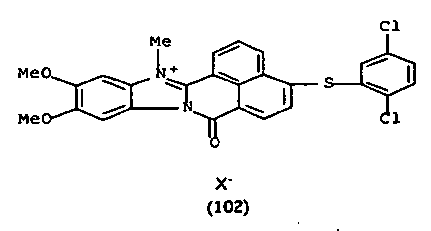

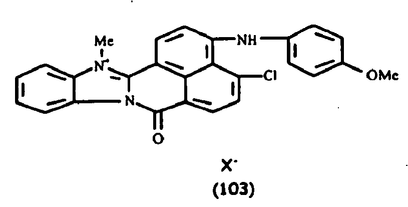

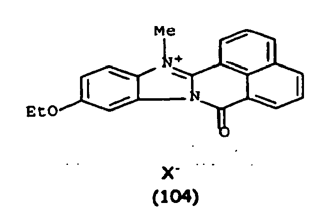

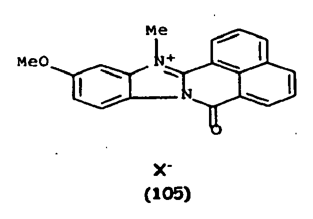

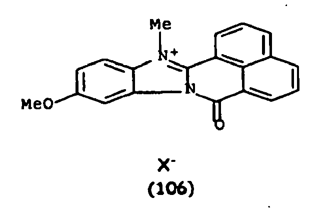

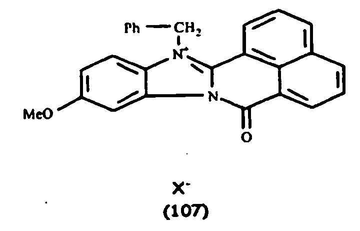

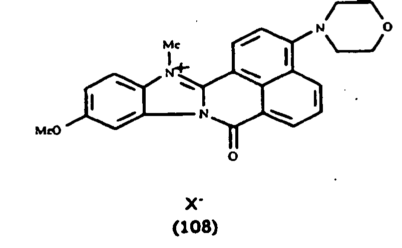

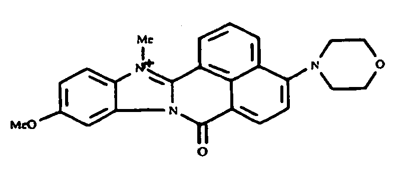

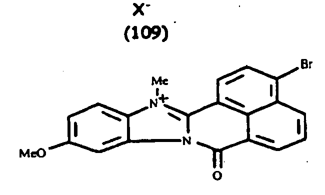

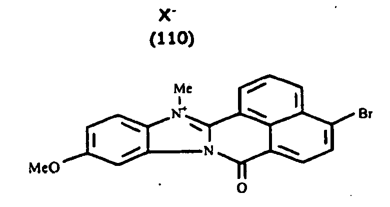

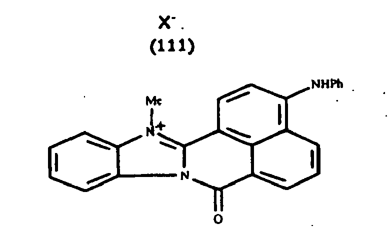

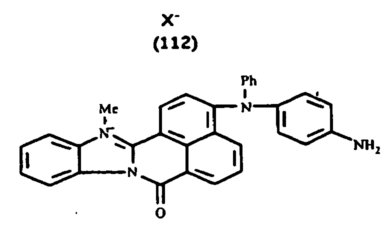

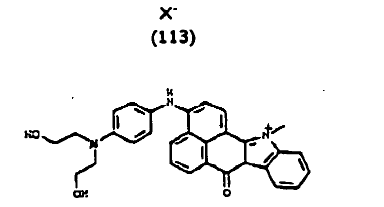

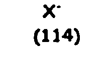

Ces composés de formule I sont des composés connus. Ils peuvent être préparés selon les méthodes décrites dans les demandes de brevet FR 2052153, CH 499601, FR 1472179, DE 2519169 et FR 1440053.

La composition selon la présente demande comprend de 0,001 à 10%, de préférence de 0,01 à 10% et encore plus préférentiellement de 0,1 à 5% en poids de colorant(s) direct(s) de formule (I) par rapport au poids total de la composition.

Selon un premier mode préféré la composition selon la présente invention contient au moins un agent de conditionnement, de préférence choisi parmi les polymères cationiques, les cations, les silicones, les chitosanes et les dérivés de chitosanes.

La concentration en agent(s) de conditionnement dans la composition selon la présente invention peut varier de 0,01 à 10% en poids par rapport au poids total de la composition, de préférence de 0,05 à 5% et plus préférentiellement encore de 0,1 à 3%.

Selon un deuxième mode préféré, la composition selon la présente invention contient au moins un polymère épaississant encore appelé "agent d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères d'acide acrylique ou d'acide acrylamidopropanesulfonique et les polymères associatifs tels que les polymères hydrosolubles capables, dans un milieu aqueux, de s'associer réversiblement entre eux ou avec d'autres molécules.

Les polymères associatifs utilisables selon l'invention peuvent être de type anionique, cationique, amphotère et de préférence non ionique.

La concentration en poids des polymères épaississants dans la composition colorante peut varier d'environ 0,01 à 10% du poids total de la composition, plus préférentiellement, cette quantité varie d'environ 0,1 à 5% en poids dans la composition colorante.

Selon un troisième mode préféré, la composition selon la présente invention contient au moins un tensioactif choisi parmi les tensioactifs anioniques, cationiques, non ioniques, amphotères ou zwitterioniques.

Les quantités d'agents tensioactifs présents dans la composition selon l'invention peuvent varier de 0,01 à 40% et de préférence de 0,1 à 30% du poids total de la composition.

Selon un quatrième mode préféré, la composition de la présente invention comprend un ou plusieurs précurseurs de colorant d'oxydation : une ou plusieurs bases d'oxydation et/ou un ou plusieurs coupleurs.

A titre d'exemple, les bases d'oxydation sont choisies parmi les phénylènediamines, les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N ,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxypyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diaminopyrazolo[1,5-a]pyridine; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine; la pyrazolo[1,5-a]pyridine-3,5-diamine ; la 5-morpholin-4-yl-pyrazolo[1,5-a]pyridin-3-ylamine; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi citer le 4,5-diamino 1β-méthoxyéthylpyrazole.

La ou les bases d'oxydation additionnelles présentes dans la composition de l'invention sont en général présentes en quantité comprise allant de 0,001 à 20 % en poids environ du poids total de la composition tinctoriale, de préférence allant de 0,005 à 6 %.

Lorsqu'au moins une base d'oxydation est présente, la composition selon l'invention contient de préférence un ou plusieurs coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs additionnels autres que les colorants directs de type naphthoylène-benzimidazolium de formule I, pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-Bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-β-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1 -naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
   ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition selon l'invention peut également contenir au moins un solvant hydroxylé, tels que notamment l'éthanol, le propylèneglycol, le glycérol, les monoéthers de polyols, l'alcool benzylique.

Elle peut aussi contenir un solvant non hydroxylé.

Les solvants hydroxylés et les solvants non hydroxylés sont, de préférence, présents dans une quantité de préférence comprise entre 1 et 40 % en poids environ, et encore plus préférentiellement entre 5 et 30 % en poids environ par rapport au poids total de la composition tinctoriale.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents régulateurs de pH, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ et de manière encore plus préférée entre 6 et 8,5. Il peut être ajusté à la valeur désirée au moyen d'agents modificateurs de pH, acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XXIII) suivante : 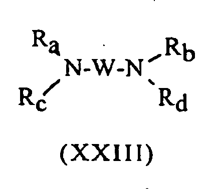 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande comprend les étapes d'appliquer la composition tinctoriale selon la présente demande sur les fibres kératiniques, puis à laisser pauser pendant une période suffisante pour permettre la coloration des cheveux, cette période est généralement comprise entre 5 minutes et 1 heure et de préférence entre 15 minutes et 1 heure.

Le procédé de teinture des fibres kératiniques, en particulier lorsque la composition selon l'invention comprend au moins un précurseur de colorant d'oxydation, peut comprendre une étape mettant en oeuvre un agent oxydant. L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention comprenant au moins un précurseur de colorant d'oxydation est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 5 minutes à 1 heure environ, de préférence 15 minutes à 1 heure environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 8,5. Il peut être ajusté à la valeur désirée au moyen d'agents régulateurs de pH, acidifiants ou alcalinisants habituellement utilisés en teinture des fibres kératiniques et tels que définis précédemment.

La composition comprenant au moins un précurseur de colorant d'oxydation, prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

L'exemple qui suit illustre l'invention sans toutefois présenter un caractère limitatif.

### Exemple de composition colorante selon l'invention :

| Composé | quantité |
|---|---|
| Iodure de 13-méthyl-7-oxo-7H-benzimidazo[2,1a]benz[de]isoquinolinium correspondant au colorant (5) | 0,2g |
| Polyquaternium 6 | 0,05g |
| Dodécylpolyglucoside | 4g MA |
| Hydroxyéthylcellulose | 0,5g |
| Ethanol | 10g |
| 2-amino 2-méthyl 1-propanol | Qs pH=9 |
| Eau déminéralisée qsp | 100 |

## Revendications

1. Utilisation pour la teinture des fibres kératiniques, en particulier des fibres kératiniques humaines telles que les cheveux, d'une composition tinctoriale comprenant dans un milieu de teinture approprié au moins un colorant direct de formule générale (I) : 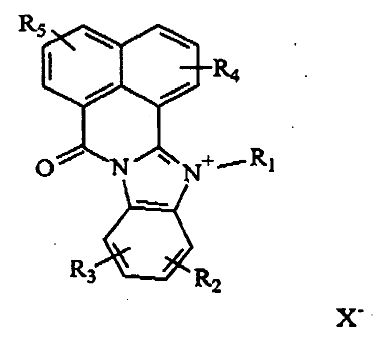 dans laquelle :
R₁ représente un groupement alkyle en C₁-C₁₀ linéaire ou ramifié, aryl alkyle en C₁-C₁₀
R₂ et R₃ représentent, indépendamment l'un de l'autre, un atome d'hydrogène ; un groupement alkyle en C₁-C₁₀ linéaire ou ramifié, un groupement alkoxy en C₁-C₁₀ linéaire ou ramifié, un atome d'halogène (F, Cl, Br, I), un groupement alcoxy en C₁-C₁₀ carbonyle ; un groupement CF₃,
R₄ et R₅ représentent, indépendamment l'un de l'autre, un atome d'hydrogène ; un atome d'halogène, un groupement alkyle en C₁-C₁₀ linéaire ou ramifié ; un groupement alkoxy en C₁-C₁₀ linéaire ou ramifié ; un groupement hydroxy, un groupement amino ; un groupement alkylamino en C₁-C₁₀ linéaire ou ramifié, un groupement dialkylamino en C₁-C₁₀ linéaire ou ramifié , un groupement phényl amino, un groupement diphényl amino, un groupement alkyle en C₁-C₄ amino phényl amino, un groupement amino phényl amino phényl, un groupement alkylthio en C₁-C₁₀ linéaire ou ratifié , un groupement phénylthio, un groupement alkyl en C₁-C₁₀ phénylthio, un groupement alkoxy en C₁-C₁₀ phénylthio, un groupement phényl alkyl en C₁-C₄ thio, morpholino, ou R₄ et R₅ forment ensemble un noyau aromatique ou hétérocyclique à 5 à 12 chaînons,
X⁻ représente un contre ion d'un acide minéral ou organique.

2. Utilisation de la composition tinctoriale selon la revendication 1 dans laquelle le colorant direct de formule générale I est tel que R₁ représente l'un des groupes suivants : alkyle en C₁-C₄; alkyle en C₁-C₅ substitué par un radical OH ; -CH₂-C₆H₄-CN ; -CH₂-C₆H₅.

3. Utilisation de la composition tinctoriale selon la revendication 1 ou 2 dans laquelle le colorant direct de formule générale I est tcl que R₂ et R₃ représentent, indépendamment l'un de l'autre, un atome d'hydrogène ; Br ; Cl ; CF₃, un groupe alkyle en C₁-C₄ ; alcoxy en C₁-C₄ ; alcoxy en C₁-C₆carbonyle, hydroxy alkyle en C₁-C₈.

4. Utilisation de la composition tinctoriale selon l'une des revendications 1 à 3 dans laquelle le colorant direct de formule général 1 est tel que R₄ et R₅ représentent indépendamment l'un de l'autre, un atome d'hydrogène, un groupement alkyle en C₁-C₄ linéaire ou ramifié ; un groupement alkyle en C₁-C₄ amino phényl amino, un groupement amino phényl amino phényl ; un groupement phénylthio, un groupement alkyl en C₁-C₁₀ phénylthio, un groupement alkoxy en C₁-C₁₀ phénylthio, un groupement phényl alkyl en C₁-C₄ thio, morpholino.

5. Utilisation de la composition tinctoriale selon l'une des revendications précédentes telle qu'elle comprend, en tant que colorant direct, au moins un composé choisi parmi les composés suivants, pour lesquels X⁻ représente un contre ion d'un acide minéral ou organique

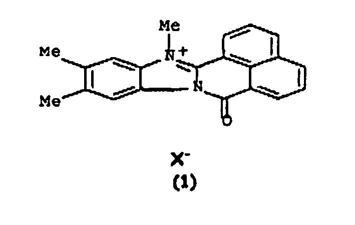

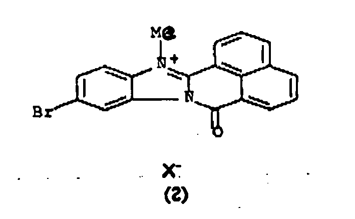

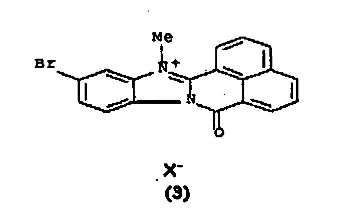

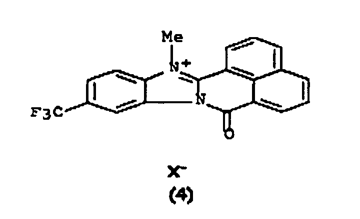

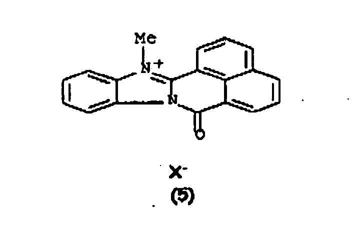

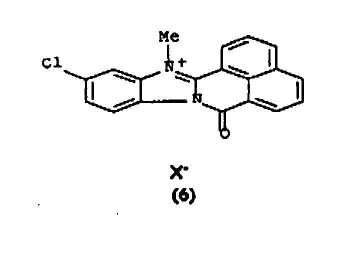

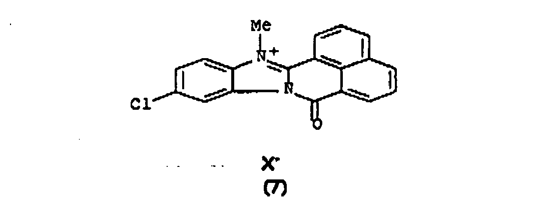

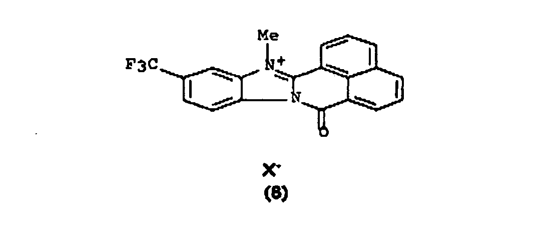

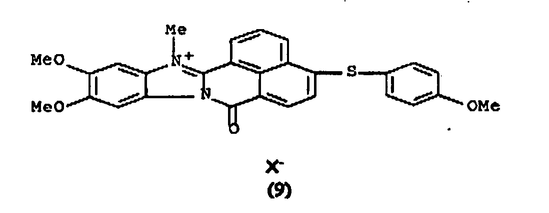

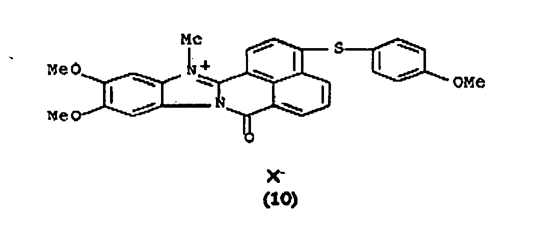

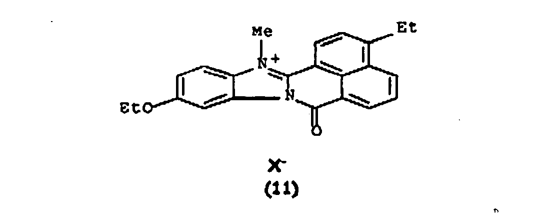

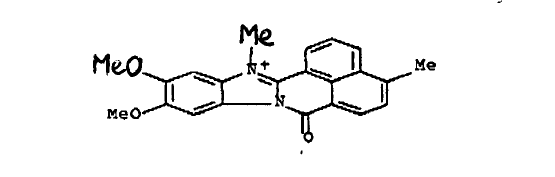

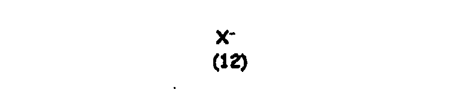

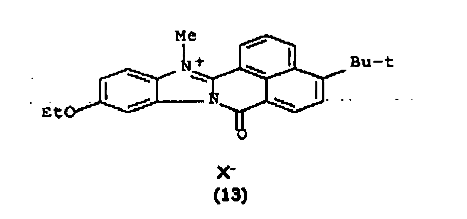

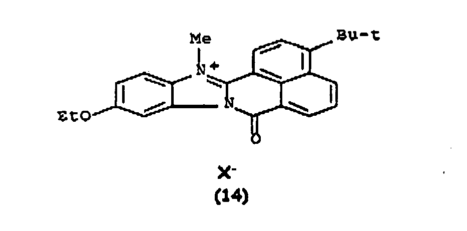

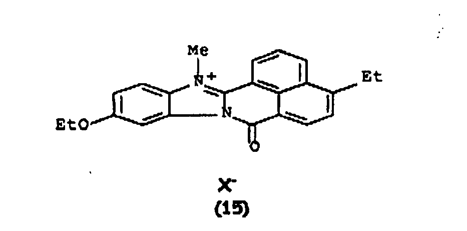

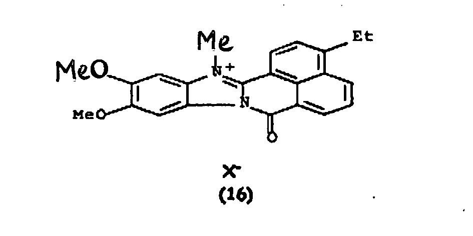

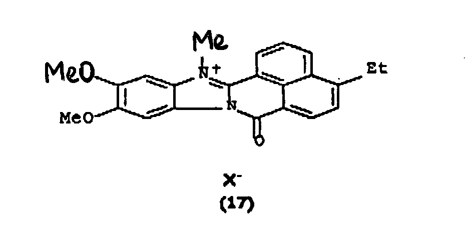

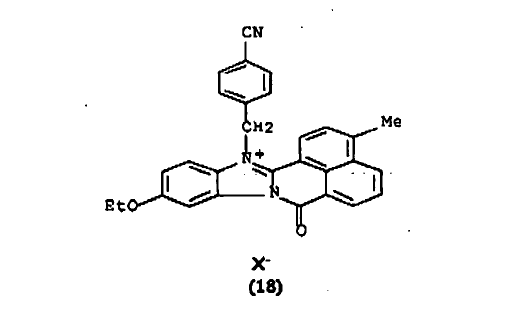

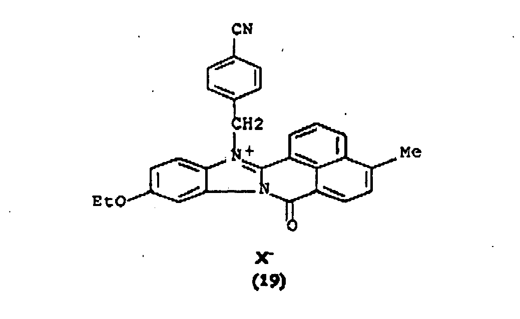

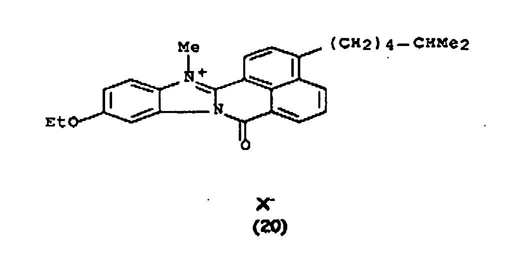

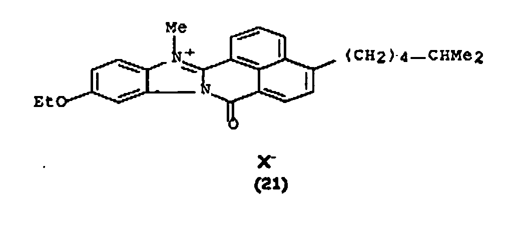

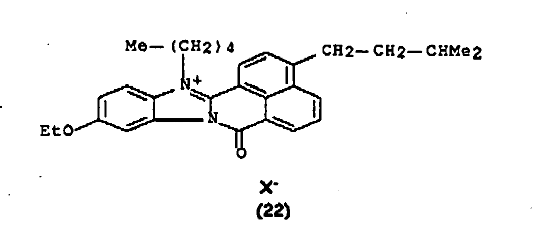

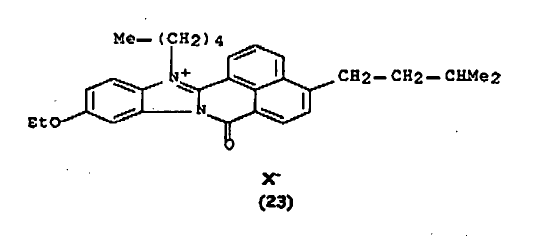

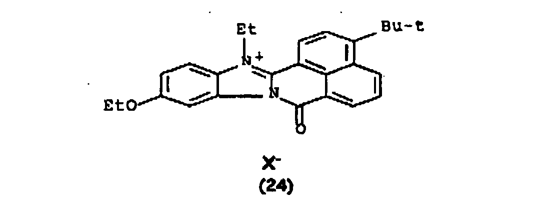

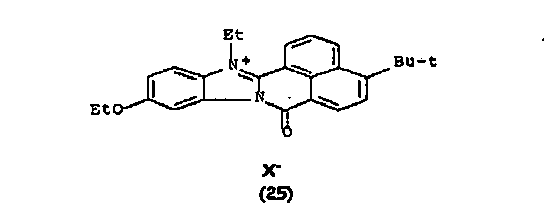

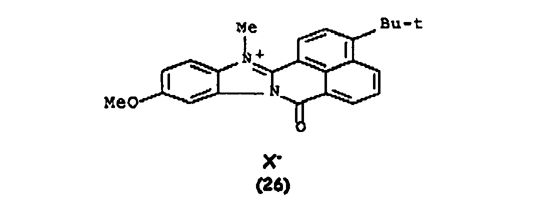

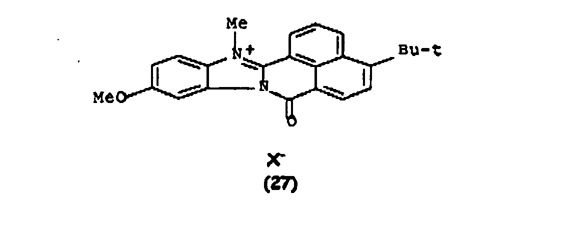

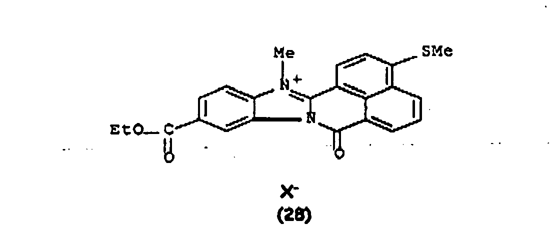

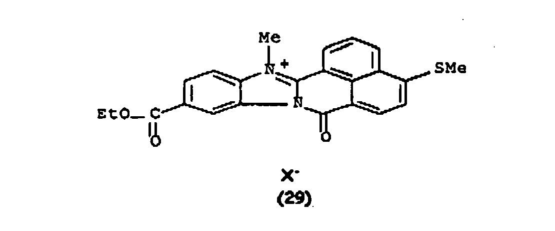

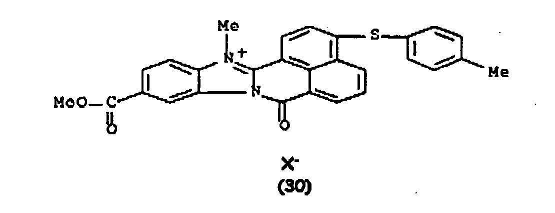

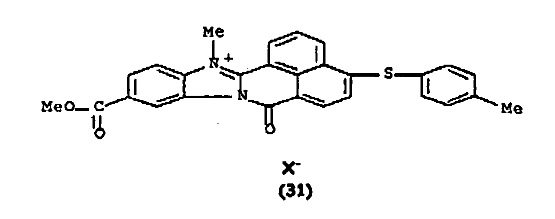

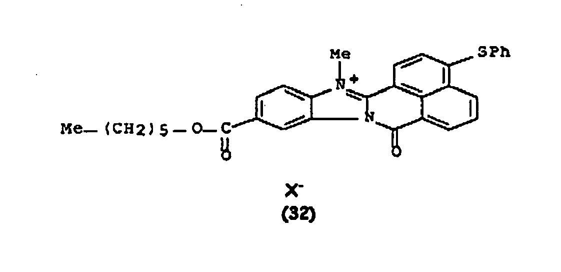

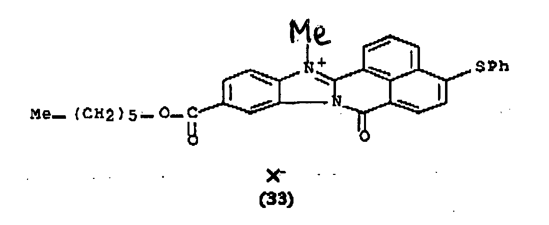

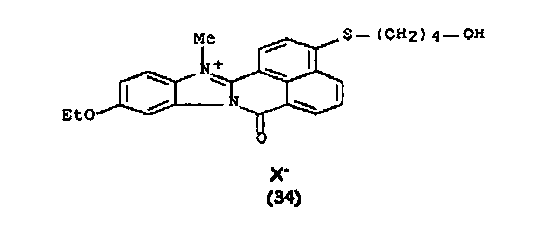

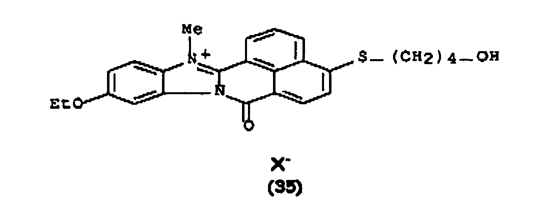

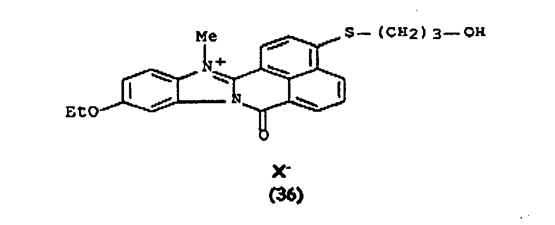

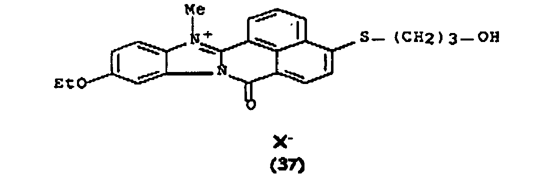

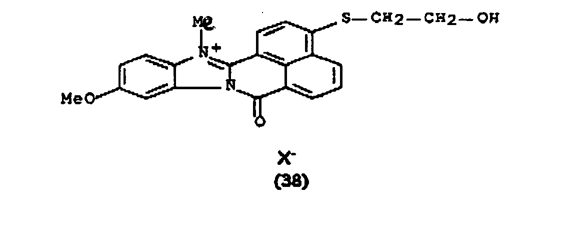

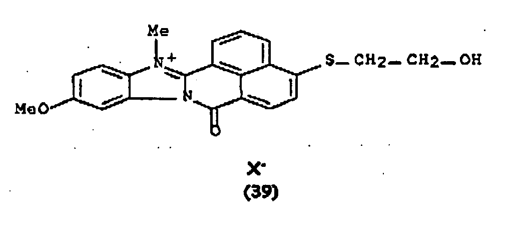

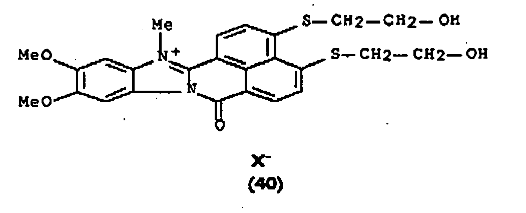

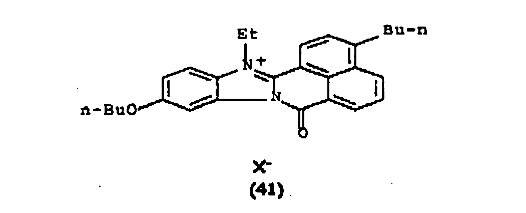

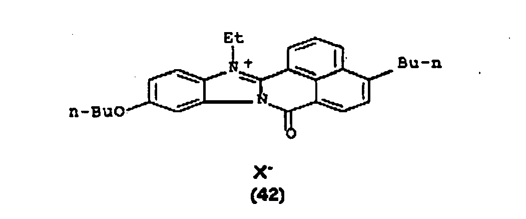

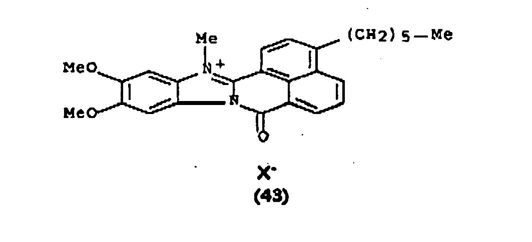

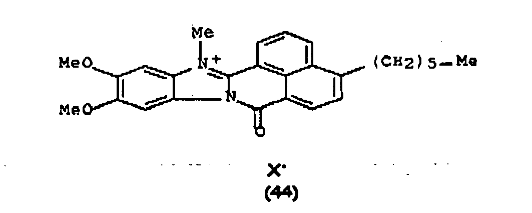

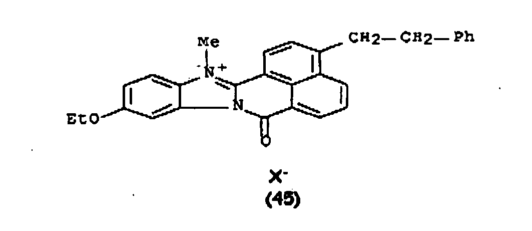

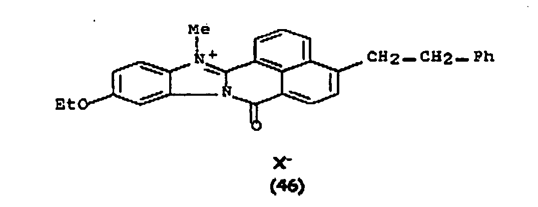

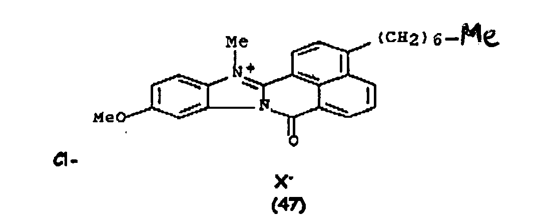

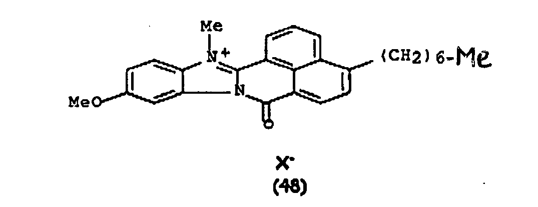

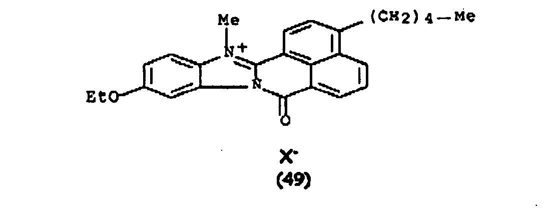

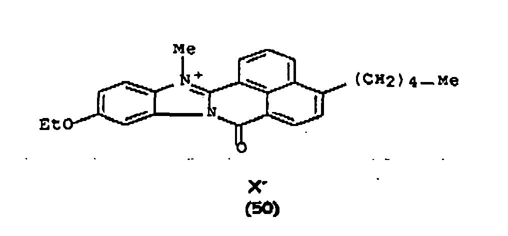

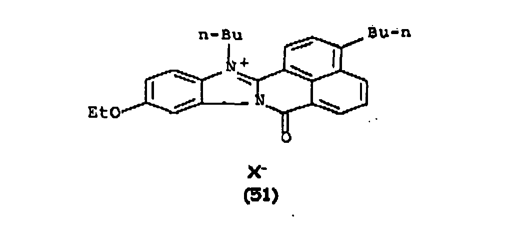

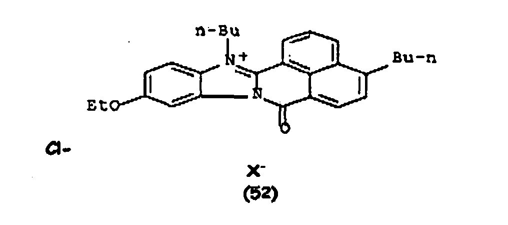

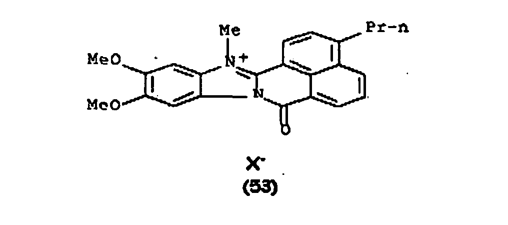

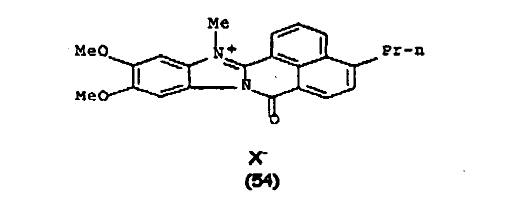

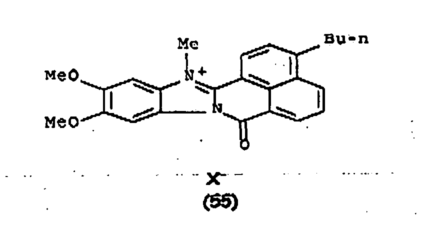

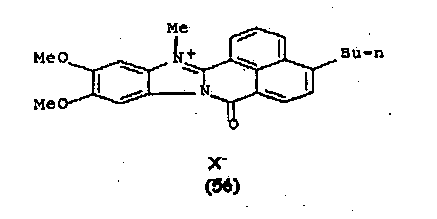

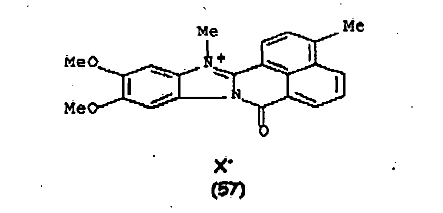

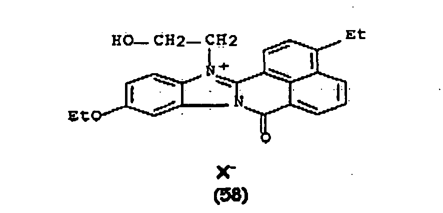

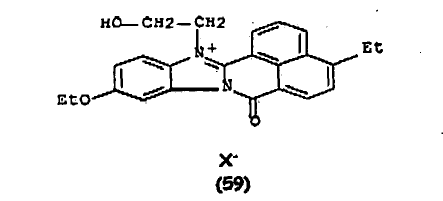

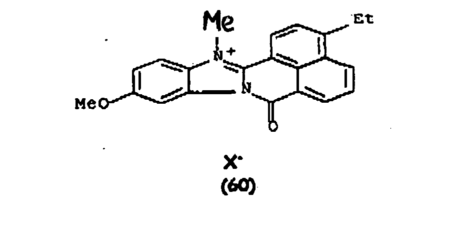

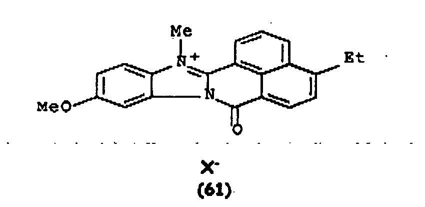

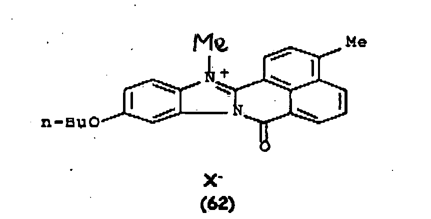

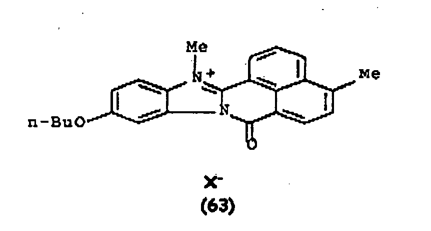

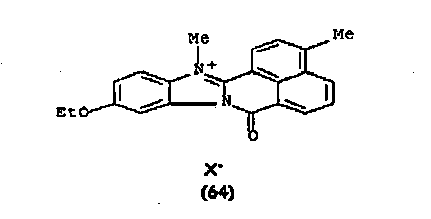

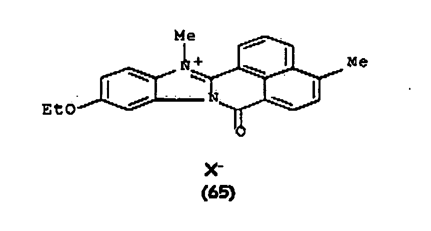

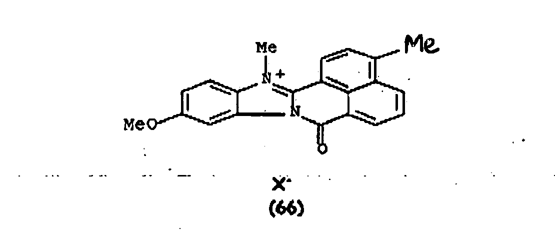

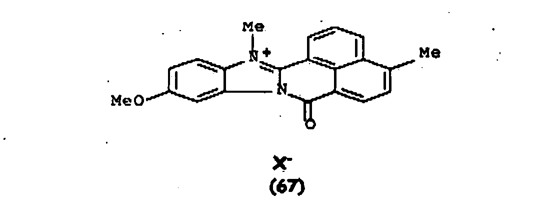

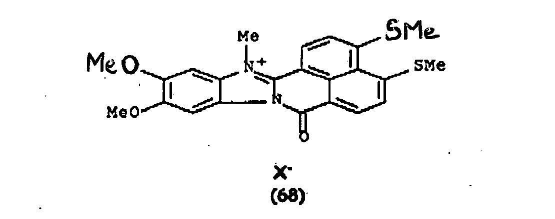

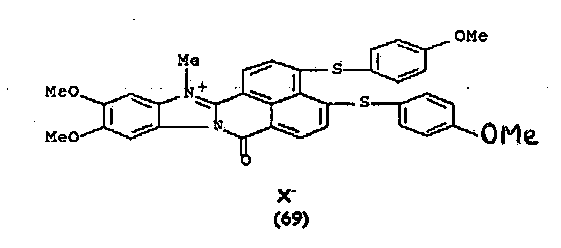

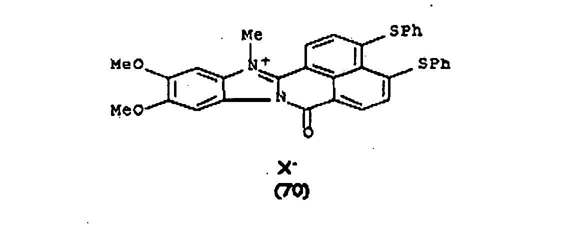

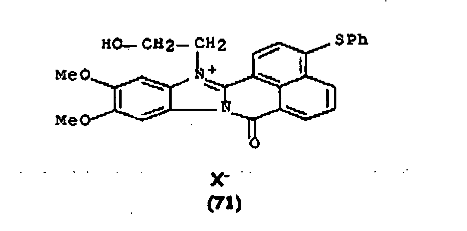

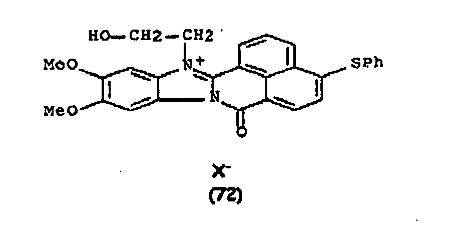

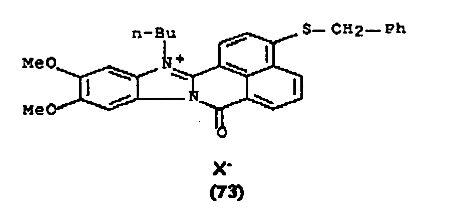

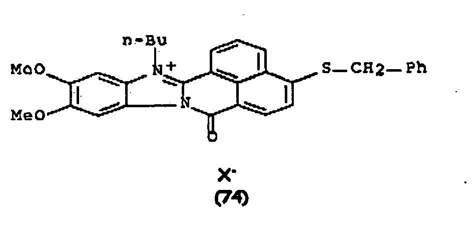

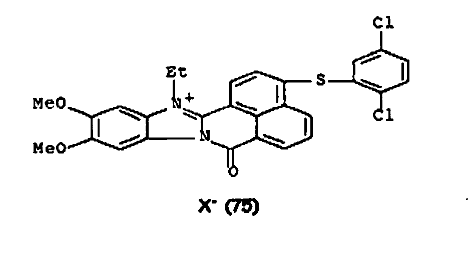

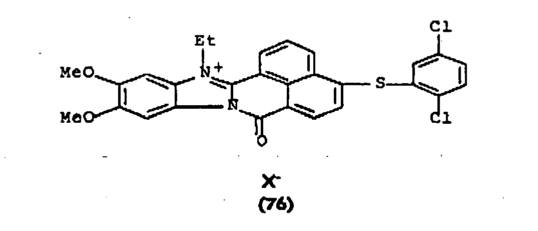

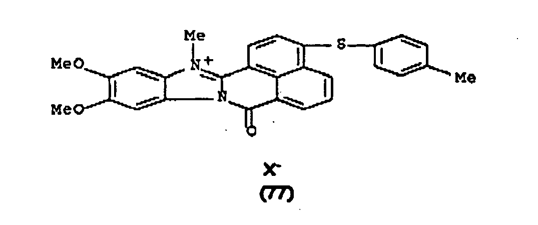

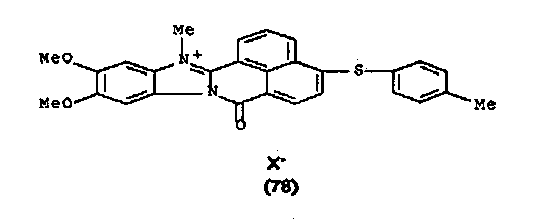

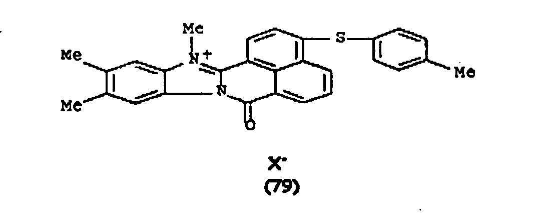

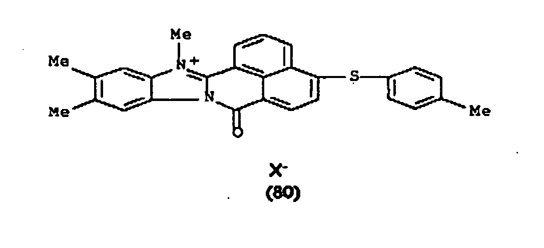

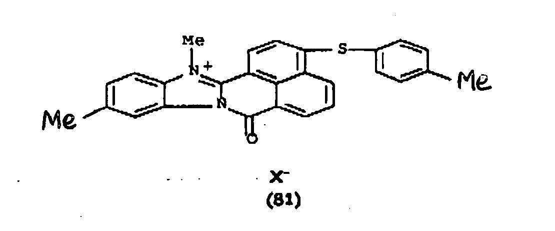

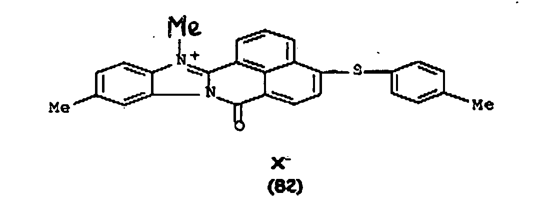

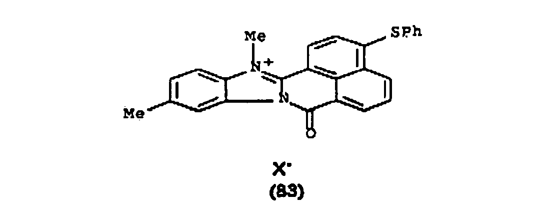

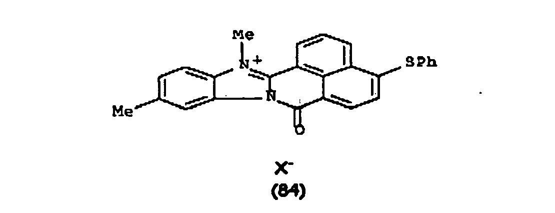

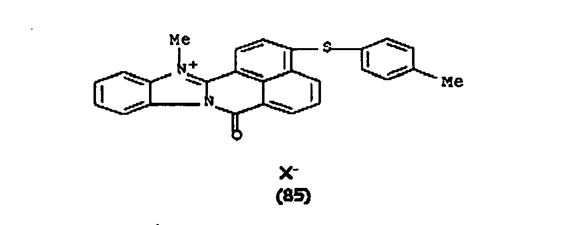

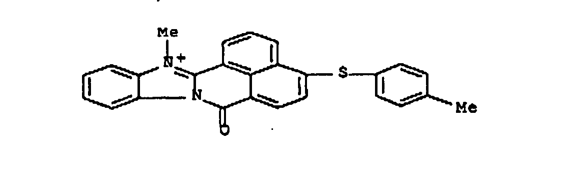

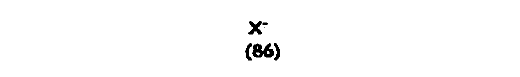

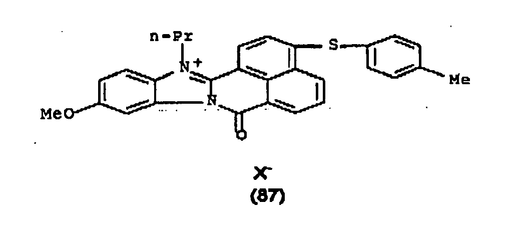

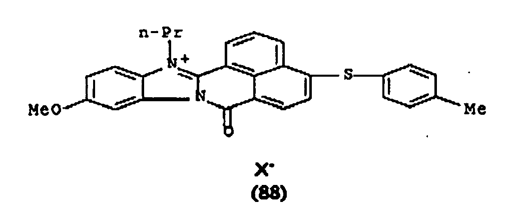

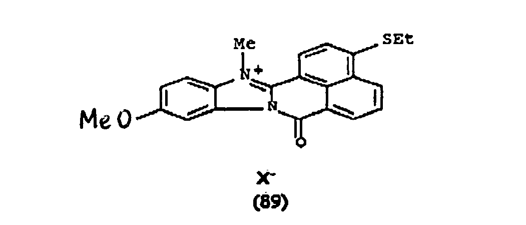

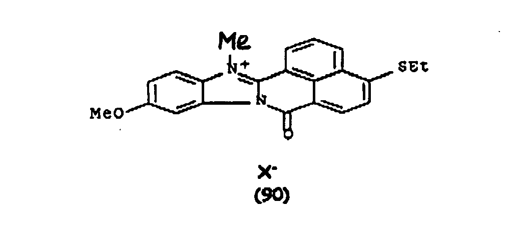

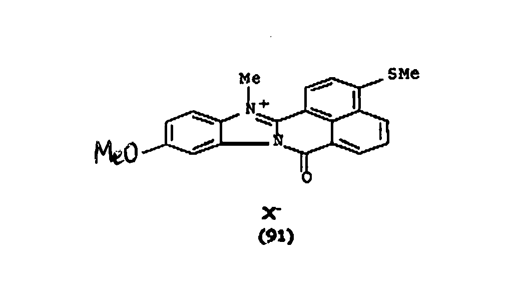

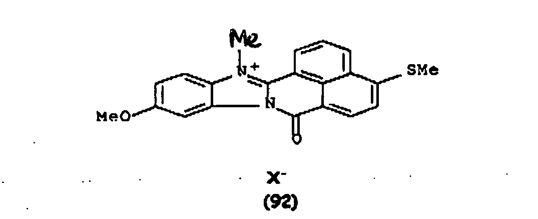

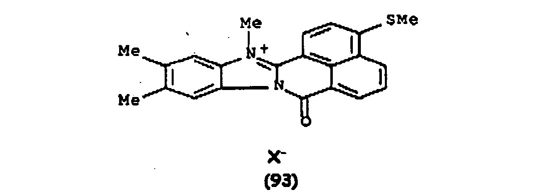

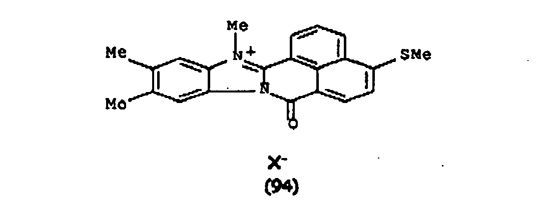

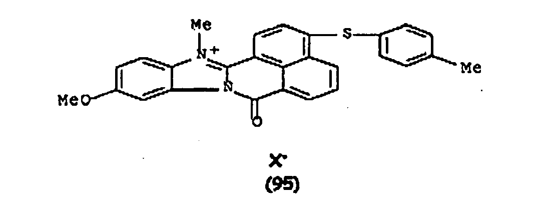

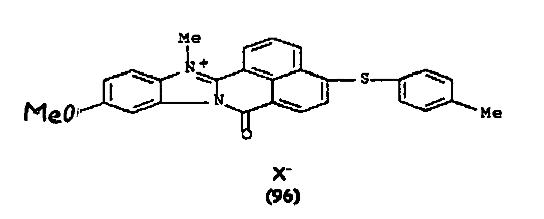

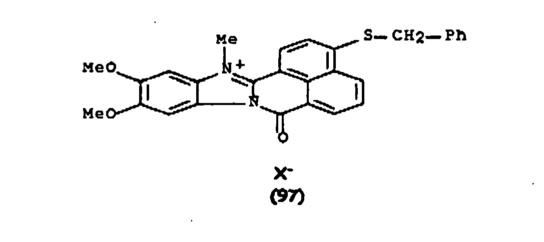

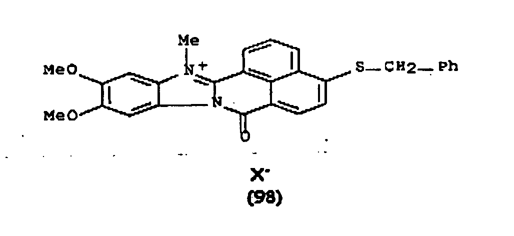

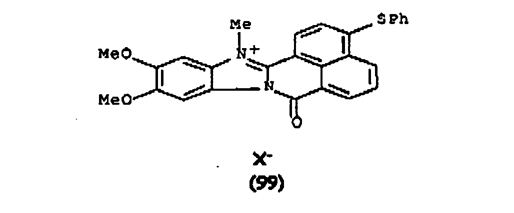

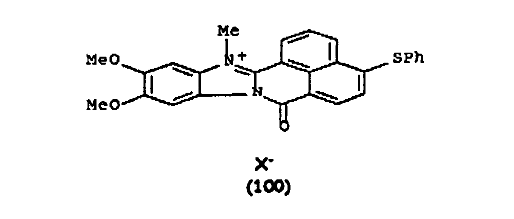

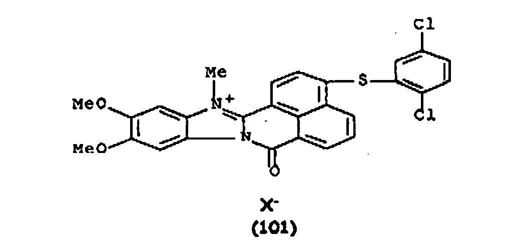

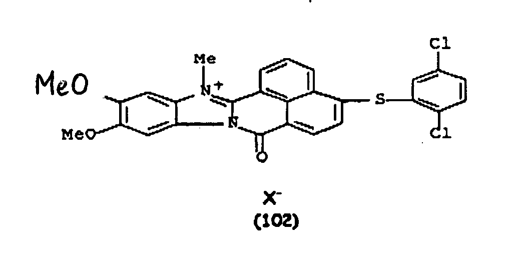

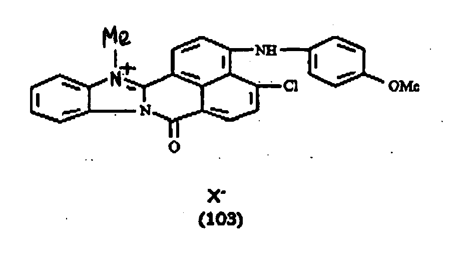

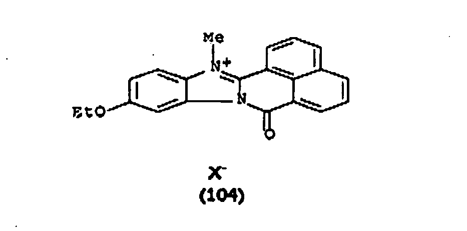

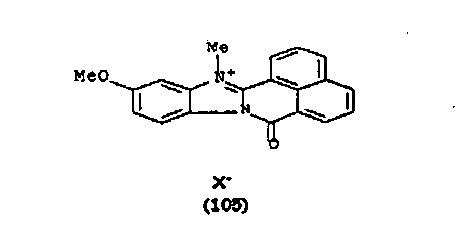

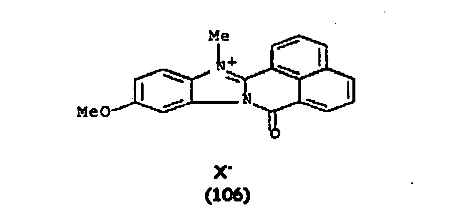

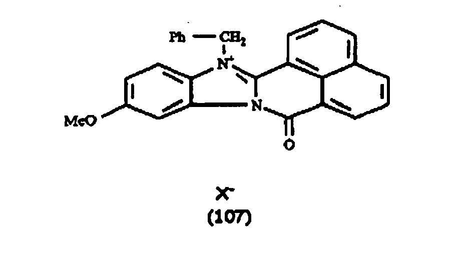

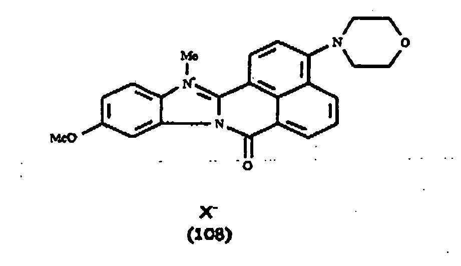

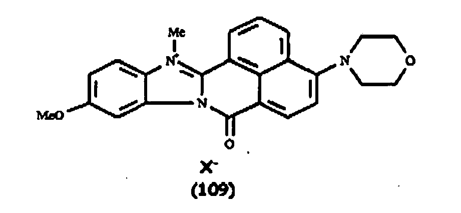

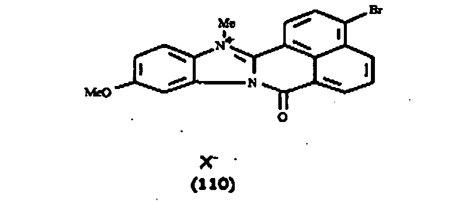

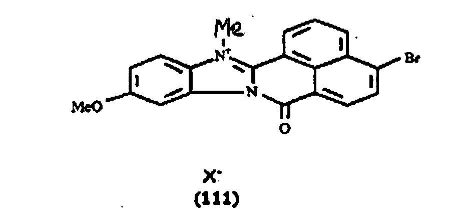

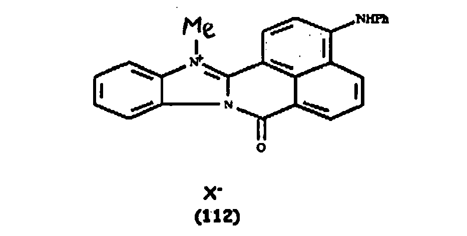

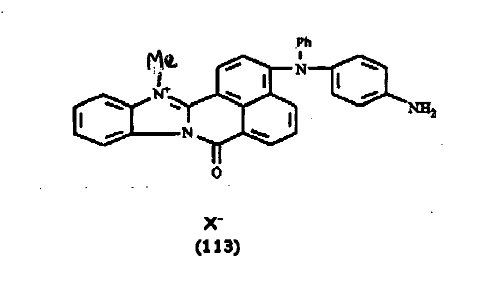

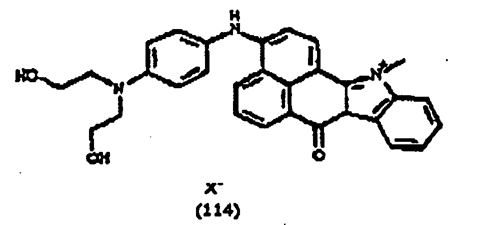

6. Composition tinctoriale pour la teinture des fibres kératiniques, en particulier des fibres kératiniques humaines telles que les cheveux, comprenant dans un milieu de teinture approprié, en plus du ou des colorants directs de formule (I) tels que définis dans l'une des revendications précédentes, au moins un précurseur de colorant d'oxydation et/ou au moins un agent de conditionnement et/ou au moins un polymère épaississant et/ou au moins un tensioactif.

7. Composition tinctoriale selon la revendication 6 telle qu'elle comprend de 0,001 à 20%, de préférence de 0,1 à 10% et de manière encore préférée de 0,1 à 5% de colorant(s) direct(s) de formule (I) par rapport au poids total de la composition.

8. Composition tinctoriale selon la revendication 6 ou 7 dans laquelle le ou les agents de conditionnement, sont choisis parmi les polymères cationiques, les cations, les silicones, les chitosanes et les dérivés de chitosane.

9. Composition tinctoriale selon l'une des revendications 6 à 8 dans laquelle le ou les tensioactifs sont choisis dans le groupe formé par les tensioactifs anioniques, les tensioactifs amphotères ou zwittérioniques, les tensioactifs non ioniques et les tensioactifs cationiques.

10. Composition tinctoriale selon l'une des revendications 6 à 9 dans laquelle le ou les précurseurs de colorant d'oxydation sont choisis parmi les bases d'oxydation et les coupleurs.

11. Composition tinctoriale selon la revendication 10 dans laquelle la ou les bases d'oxydation sont choisies parmi les paraphénylènediamines, les bis-phénylalkylènediamines, les paraaminophénols, les orthoaminophénols, les bases hétérocycliques et leurs sels d'addition.

12. Composition tinctoriale selon la revendication 10 ou 11, dans laquelle la ou les bases d'oxydation sont présentes en une quantité comprise entre 0,001 à 20 % en poids et de préférence entre 0,005 et 6 % en poids par rapport au poids total de la composition.

13. Composition tinctoriale selon l'une des revendications 10 à 12 dans laquelle le ou les coupleurs sont choisis parmi les métaphénylènediamines, les métaaminophénols, les métadiphénols, les coupleurs naphtaléniques, les coupleurs hétérocycliques et leurs sels d'addition.

14. Composition selon la revendication 13 dans laquelle le ou les coupleurs sont choisis parmi le 1,3-dihydroxy benzène, le 1,3-dihydroxy 2-méthyl benzène, le 4-chloro 1,3-dihydroxy benzène, le 2,4-diamino 1-(B-hydroxyéthyloxy) benzène, le 2-amino 4-(B-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-8-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

15. Composition selon la revendication 13 ou 14 dans laquelle le ou les coupleurs sont présents en une quantité comprise entre 0,001 et 20 % de préférence entre 0,005 et 6 % en poids par rapport au poids total de la composition.

16. Composition selon l'une des revendications précédentes telle qu'elle comprend au moins un colorant direct additionnel.

17. Composition selon l'une des revendications précédentes telle qu'elle comprend au moins un solvant hydroxylé tel que l'éthanol, le propylène glycol, le glycérol, les mono éthers de polyols.

18. Composition selon l'une des revendications précédentes telle qu'elle comprend au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

19. Procédé de teinture des fibres kératiniques, tel qu'il comprend les étapes d'appliquer la composition tinctoriale selon l'une des revendications 6 à 18 sur les fibres kératiniques, puis à laisser pauser pendant une période comprise entre 5 minutes et 1 heure et de préférence entre 15 minutes et 1 heure.

20. Utilisation de la composition selon l'une des revendications 6 à 18 pour l'obtention sur les fibres kératiniques, en particulier les fibres kératiniques humaines telles que les cheveux de nuances très puissantes, peu sélectives et tenaces.

## Claims

1. Use for dyeing keratin fibres, in particular human keratin fibres such as the hair, of a dye composition comprising, in a suitable dyeing medium, at least one direct dye of general formula (I): 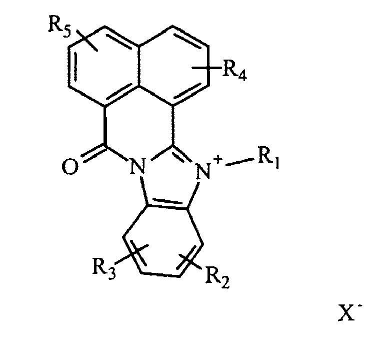 in which:
R₁ represents a linear or branched C₁-C₁₀ alkyl or aryl(C₁-C₁₀)alkyl group,
R₂ and R₃ represent, independently of each other, a hydrogen atom; a linear or branched C₁-C₁₀ alkyl group, a linear or branched C₁-C₁₀ alkoxy group, a halogen atom (F, Cl, Br or I), a (C₁-C₁₀)alkoxycarbonyl group; a CF₃ group,
R₄ and R₅ represent, independently of each other, a hydrogen atom; a halogen atom, a linear or branched C₁-C₁₀ alkyl group; a linear or branched C₁-C₁₀ alkoxy group; a hydroxyl group, an amino group; a linear or branched C₁-C₁₀ alkylamino group, a linear or branched C₁-C₁₀ dialkylamino group, a phenylamino group, a diphenylamino group, a (C₁-C₄)alkylaminophenylamino group, an aminophenylaminophenyl group; a linear or branched C₁-C₁₀ alkylthio group, a phenylthio group, a (C₁-C₁₀)alkylphenylthio group, a (C₁-C₁₀)alkoxyphenylthio group, a phenyl(C₁-C₄)alkylthio group or morpholino, or R₄ and R₅ together form a 5- to 12-membered aromatic or heterocyclic nucleus,
X- represents a counterion of a mineral or organic acid.

2. Use of the dye composition according to Claim 1, in which the direct dye of general formula I is such that R₁ represents one of the following groups: C₁-C₄ alkyl; C₁-C₅ alkyl substituted with an OH radical; -CH₂-C₆H₄-CN; -CH₂-C₆H₅.

3. Use of the dye composition according to Claim 1 or 2, in which the direct dye of general formula I is such that R₂ and R₃ represent, independently of each other, a hydrogen atom; Br; Cl; CF₃, a C₁-C₄ alkyl group; C₁-C₄ alkoxy; (C₁-C₆)alkoxycarbonyl, C₁-C₈ hydroxyalkyl.

4. Use of the dye composition according to one of Claims 1 to 3, in which the direct dye of general formula I is such that R₄ and R₅ represent, independently of each other, a hydrogen atom, a linear or branched C₁-C₄ alkyl group; a (C₁-C₄)alkylaminophenylamino group, an aminophenylaminophenyl group; a phenylthio group, a (C₁-C₁₀) alkylphenylthio group, a (C₁-C₁₀) alkoxyphenylthio group, a phenyl(C₁-C₄)alkylthio group or morpholino.

5. Use of the dye composition according to one of the preceding claims, such that it comprises, as direct dye, at least one compound chosen from the following compounds, for which X⁻ represents a counterion of a mineral or organic acid

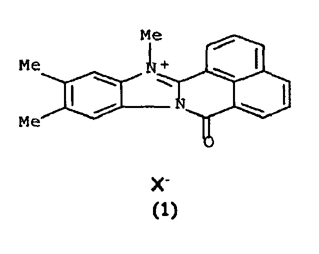

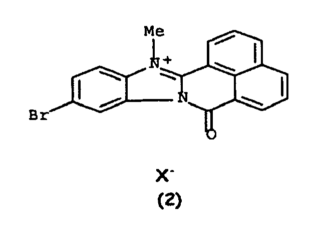

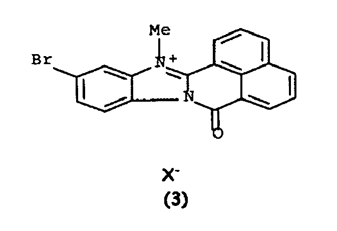

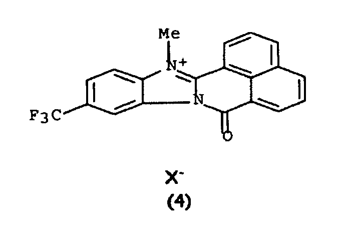

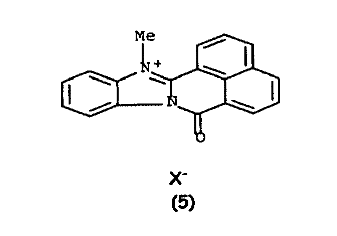

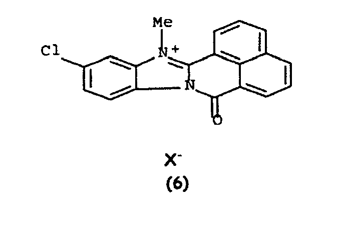

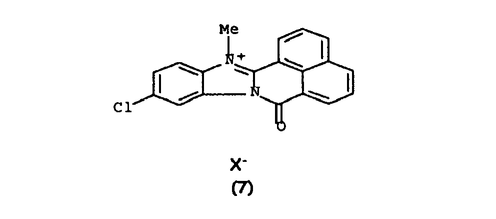

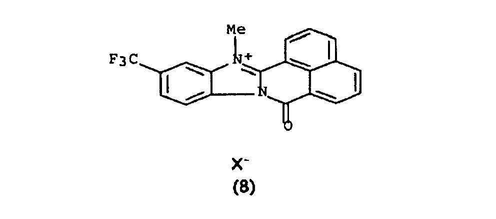

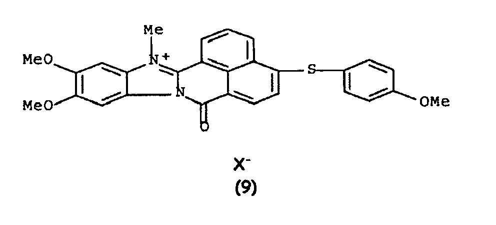

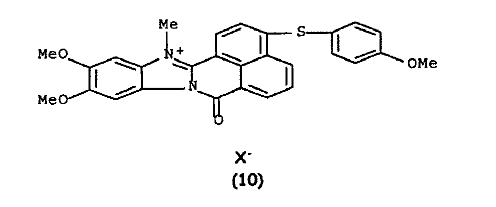

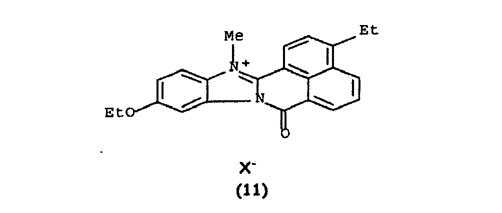

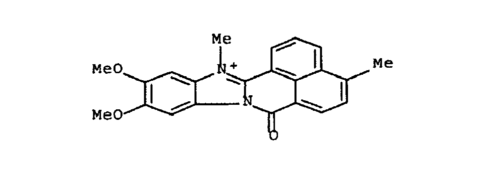

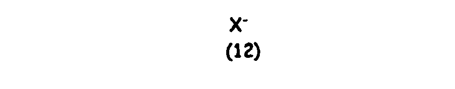

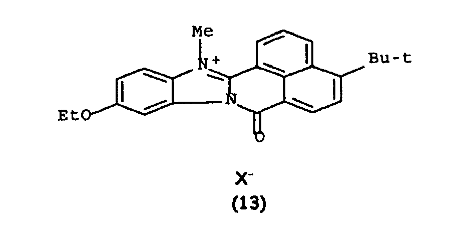

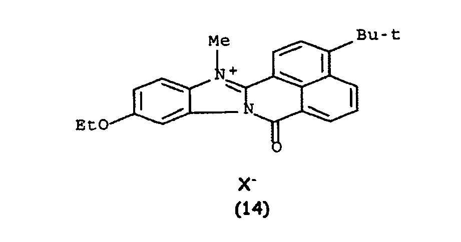

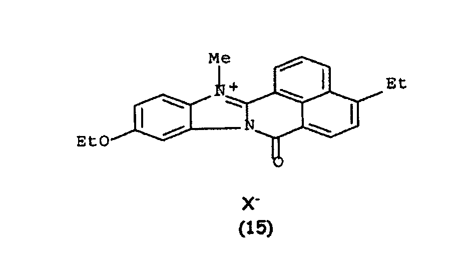

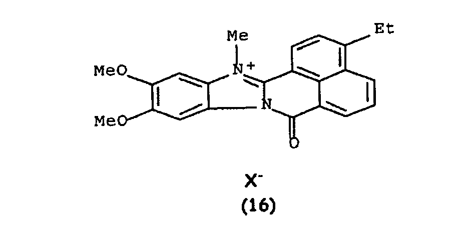

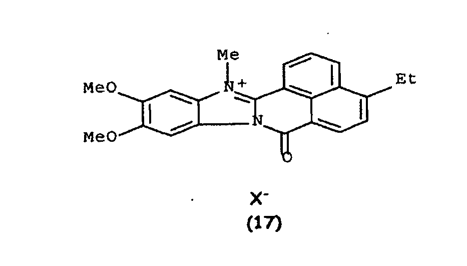

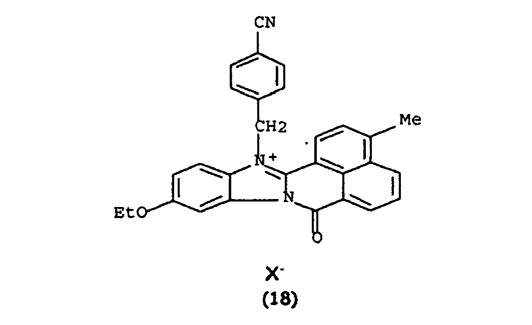

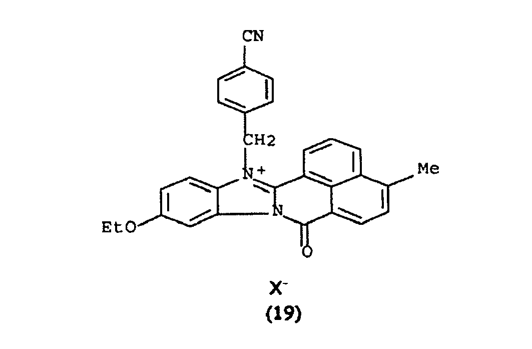

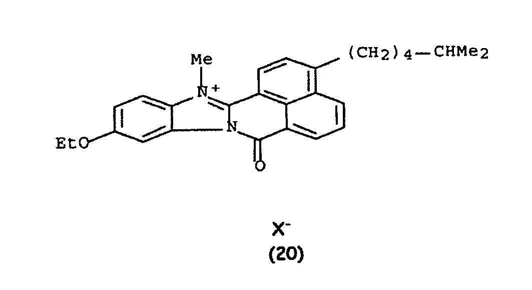

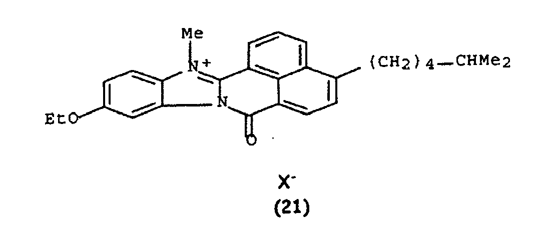

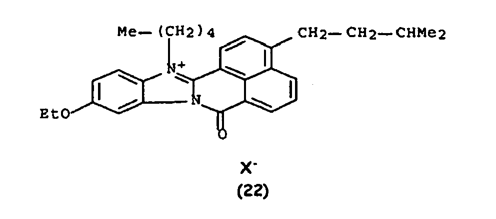

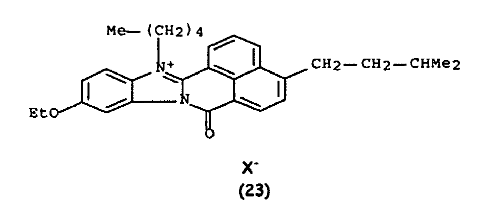

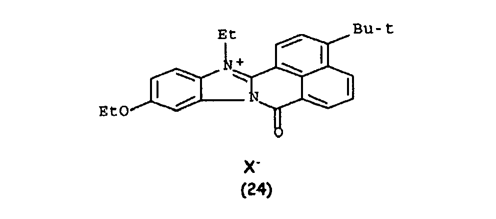

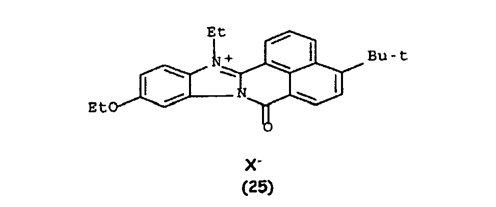

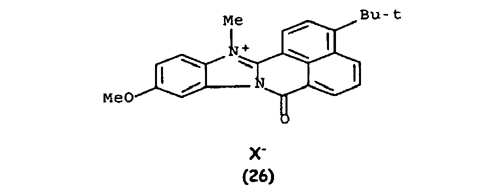

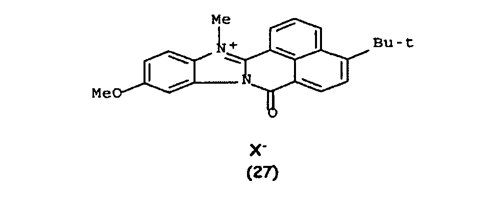

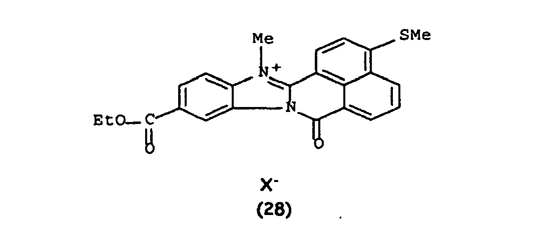

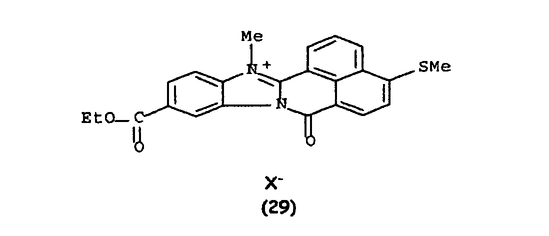

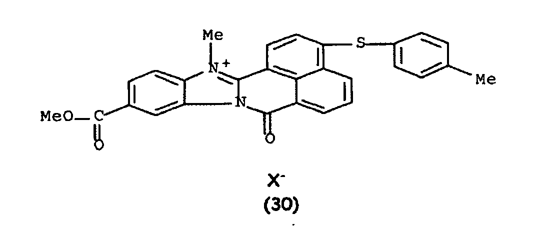

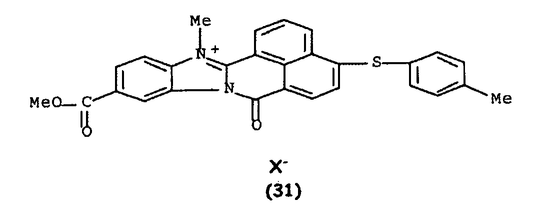

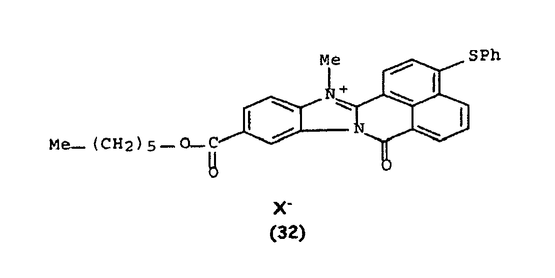

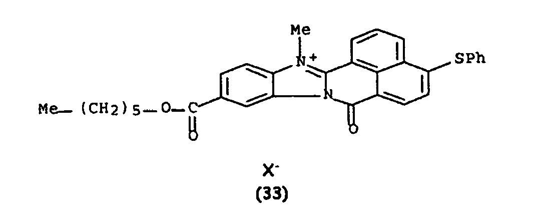

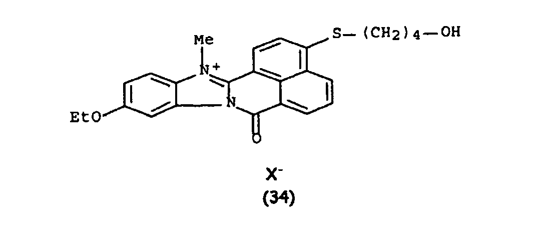

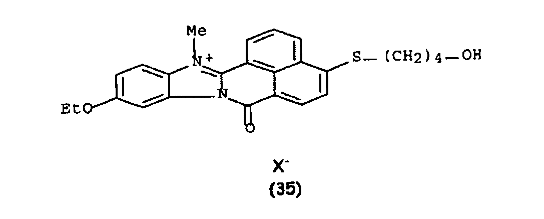

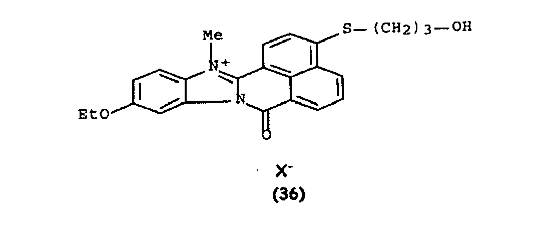

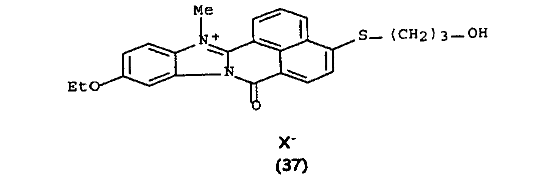

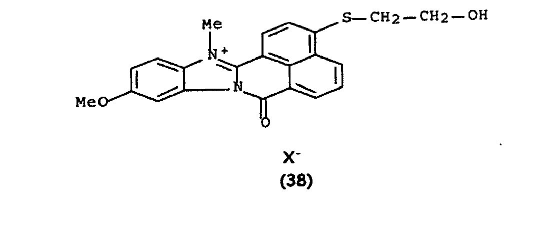

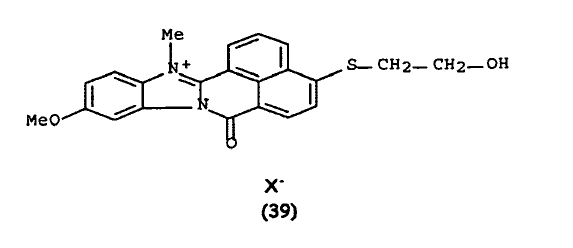

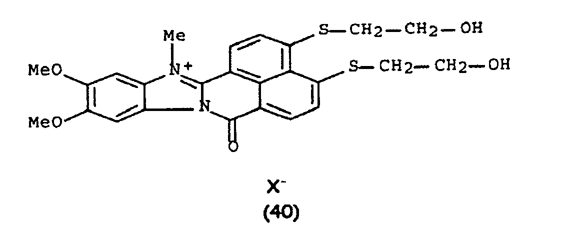

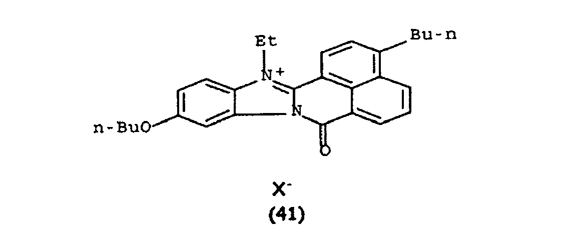

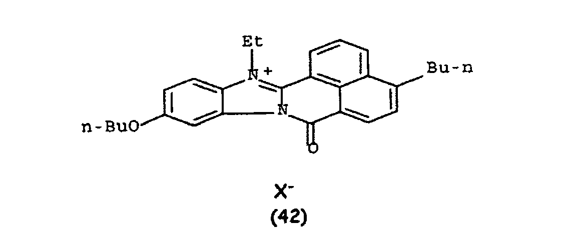

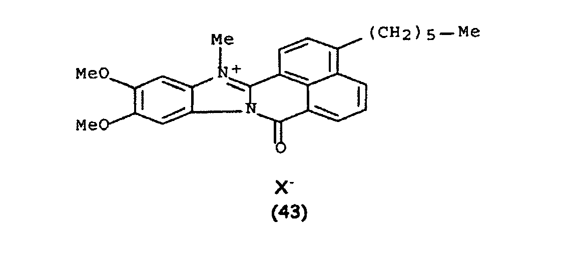

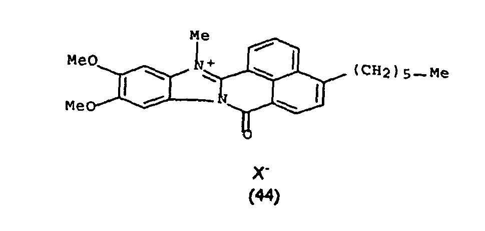

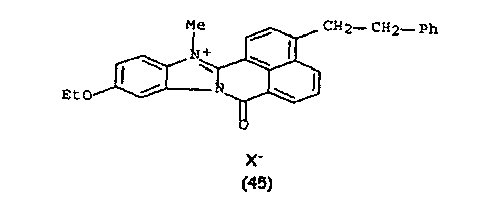

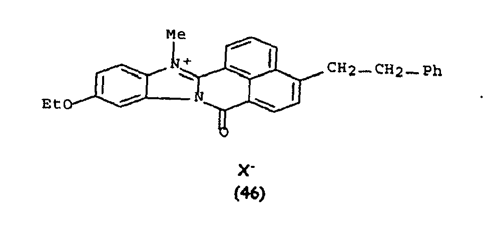

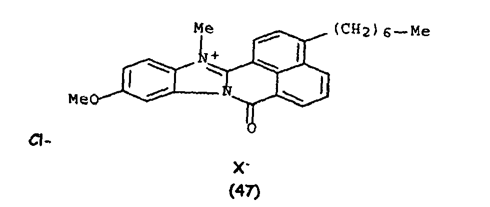

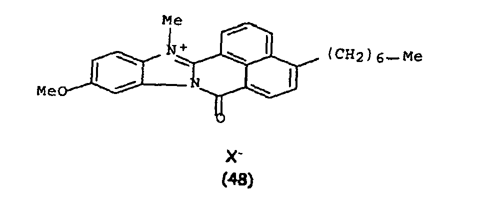

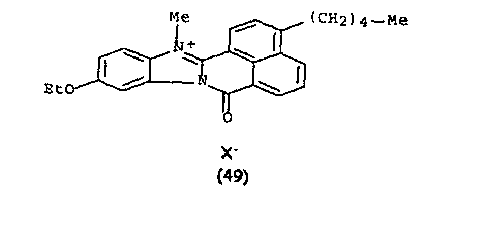

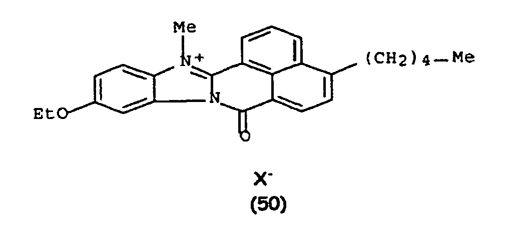

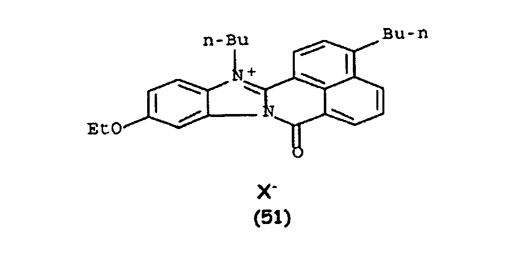

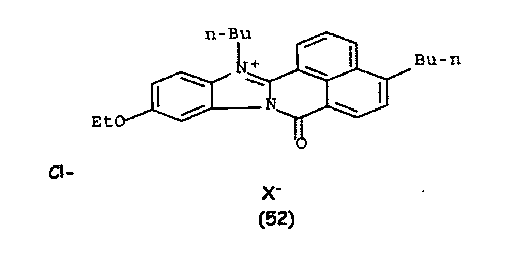

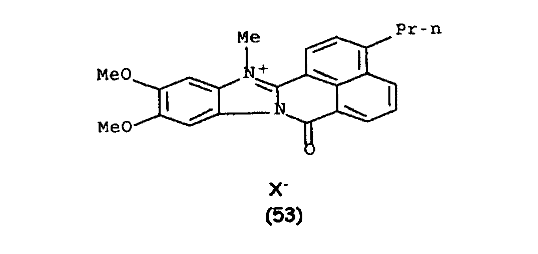

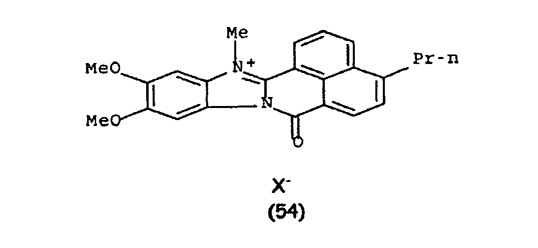

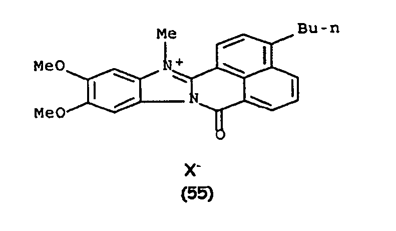

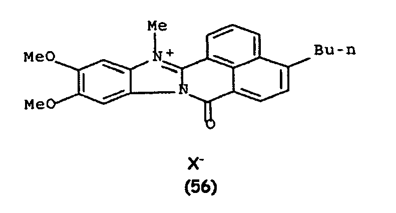

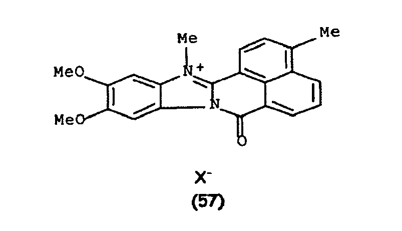

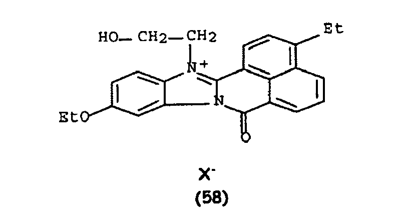

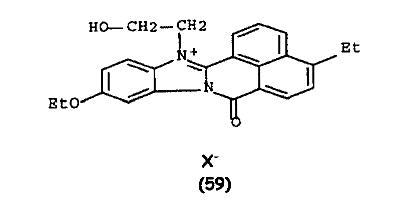

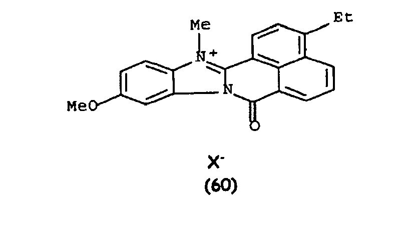

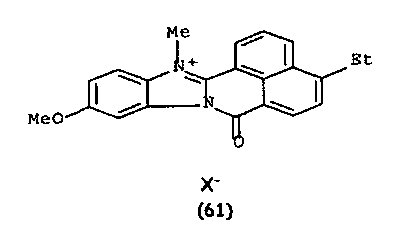

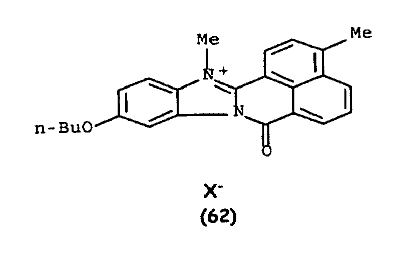

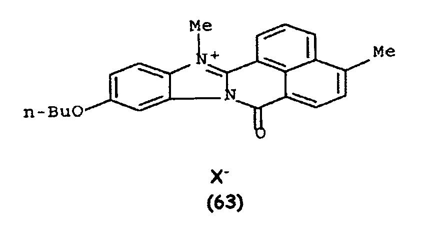

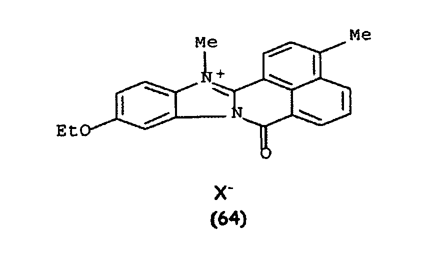

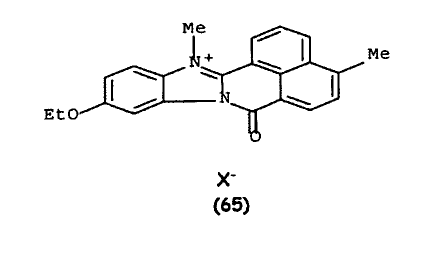

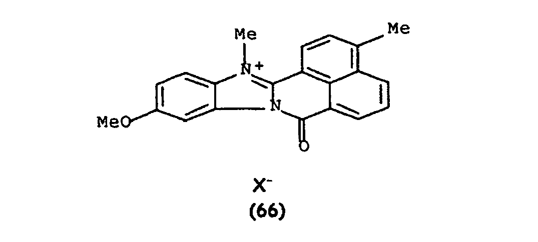

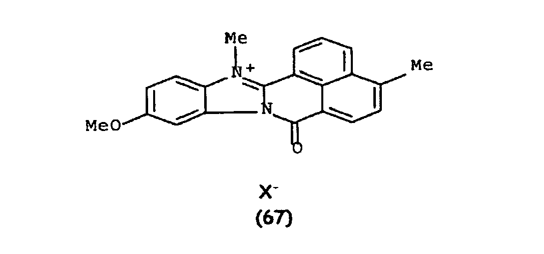

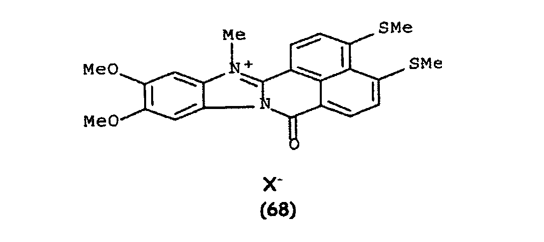

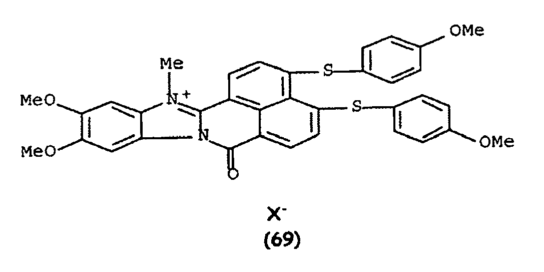

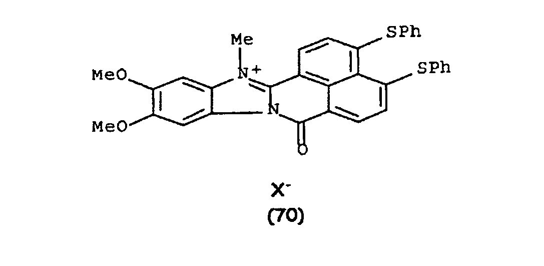

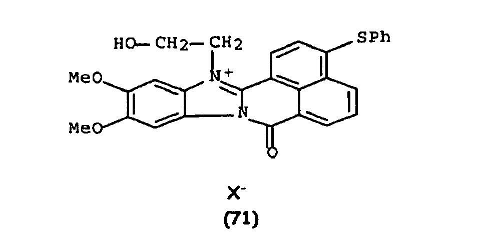

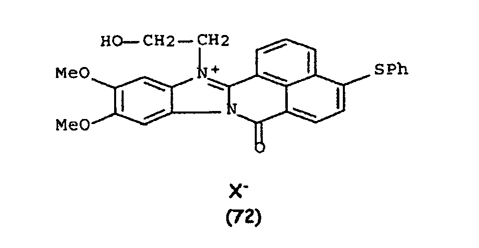

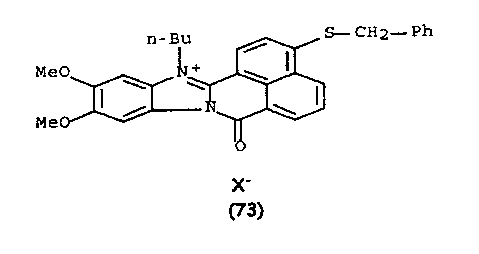

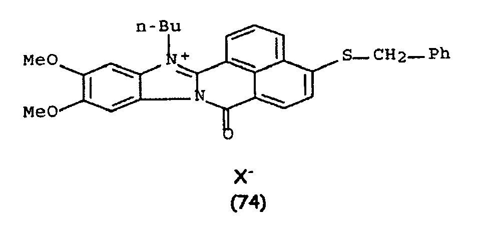

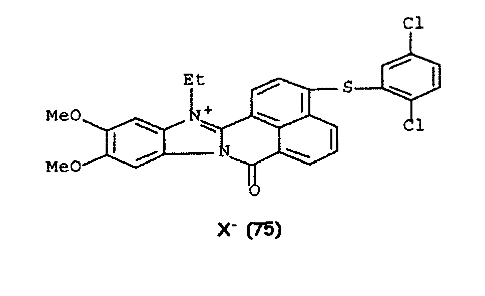

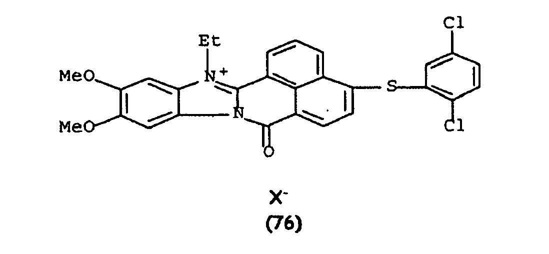

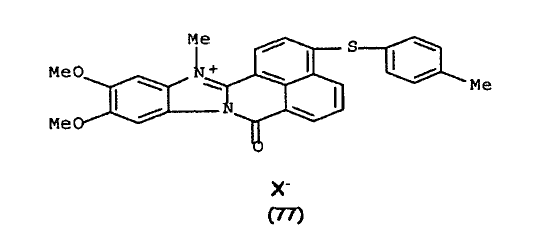

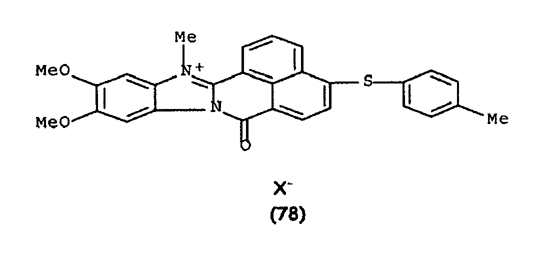

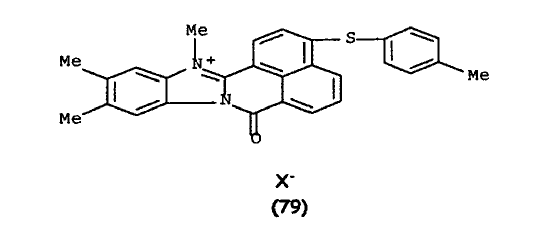

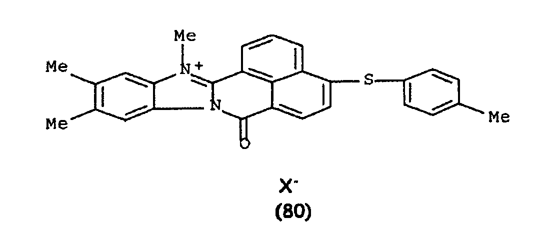

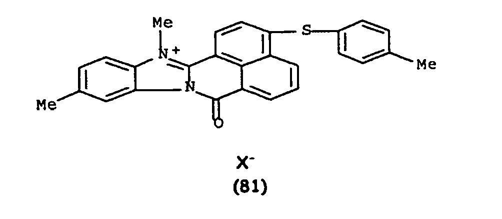

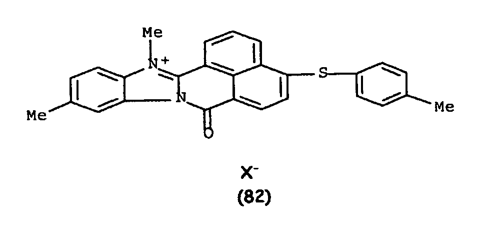

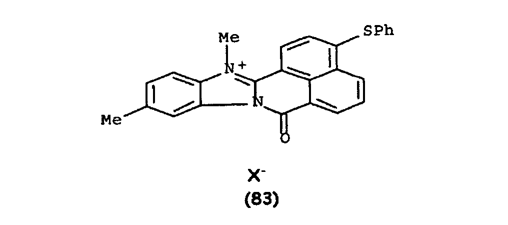

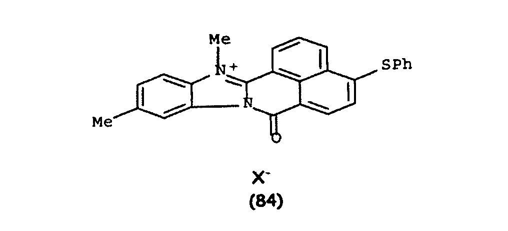

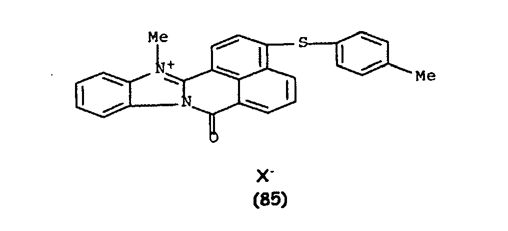

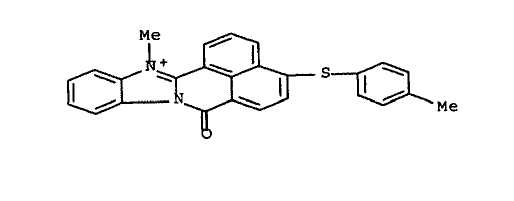

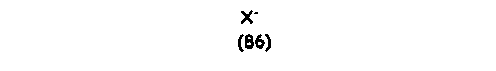

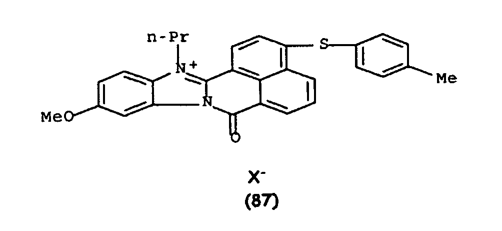

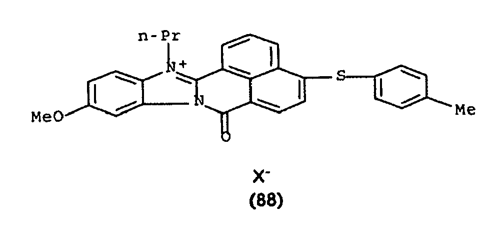

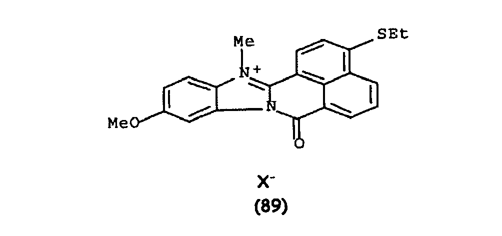

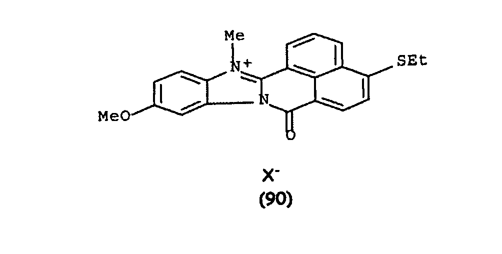

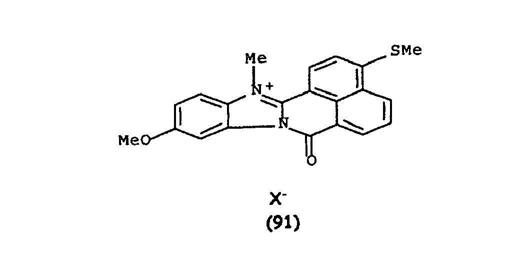

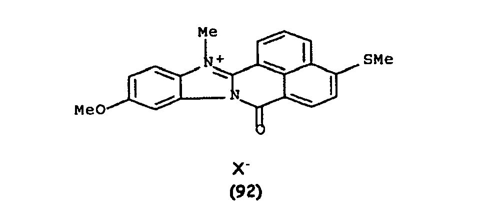

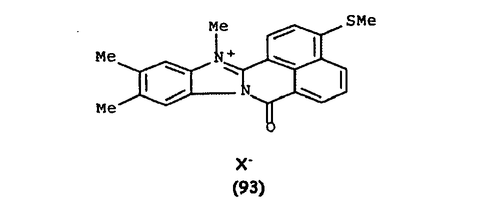

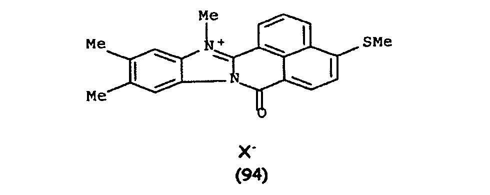

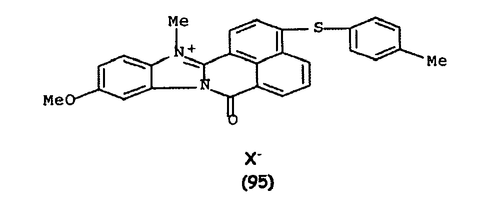

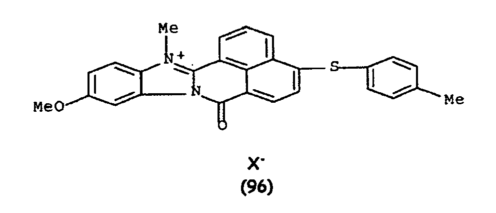

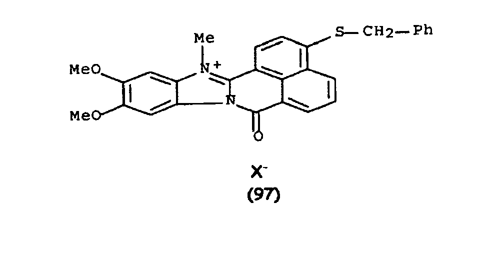

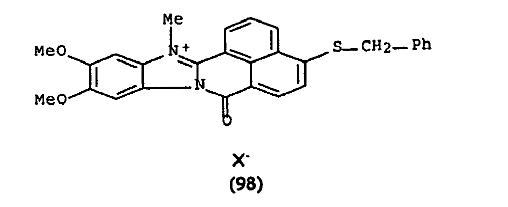

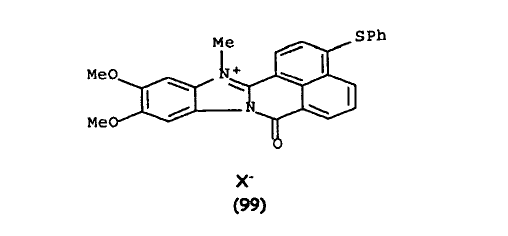

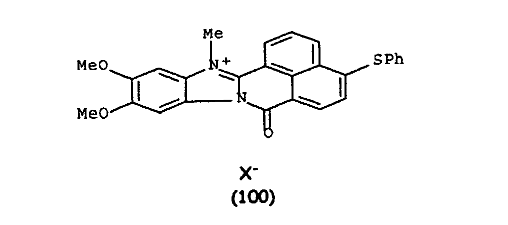

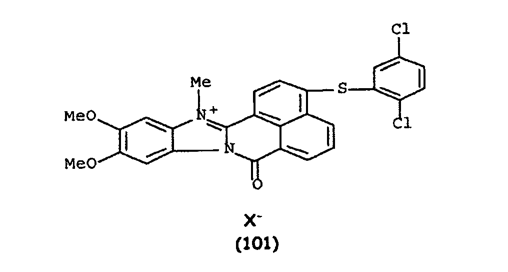

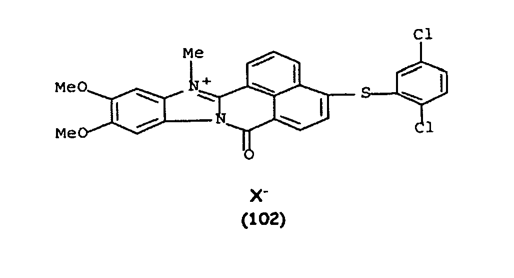

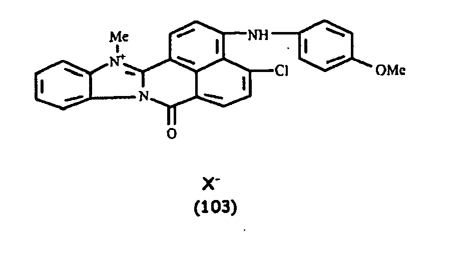

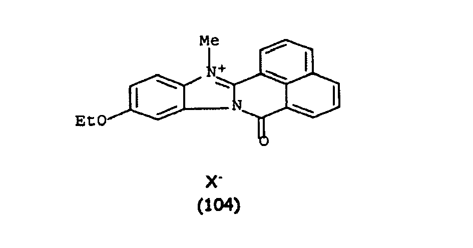

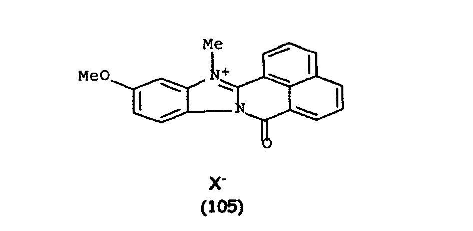

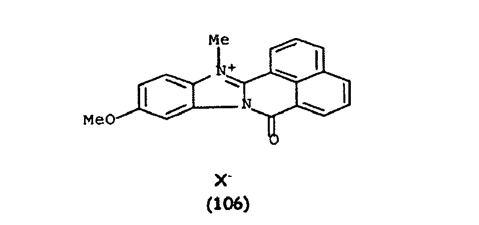

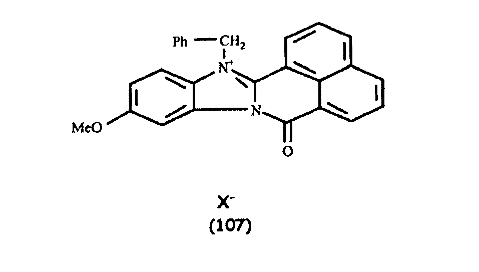

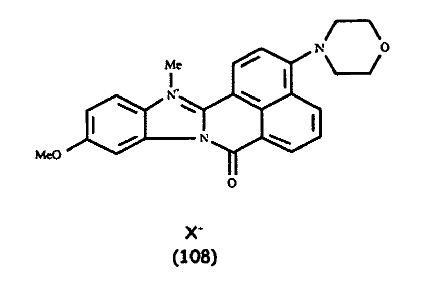

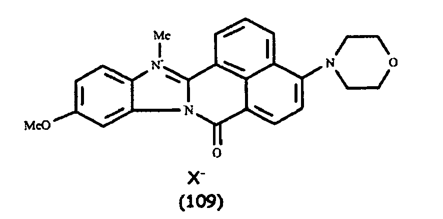

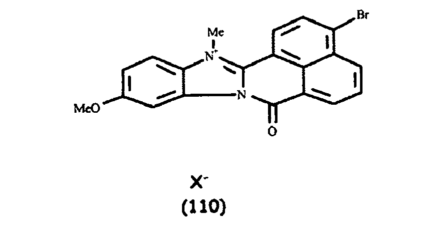

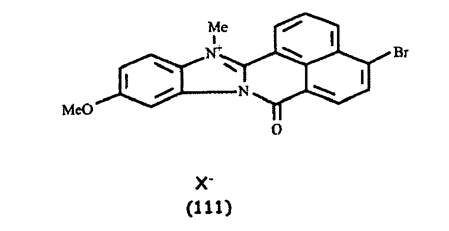

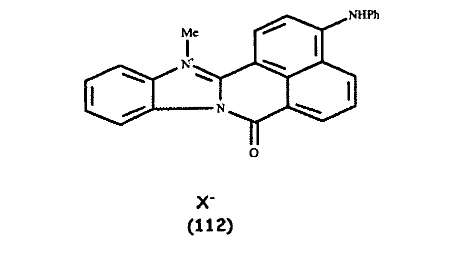

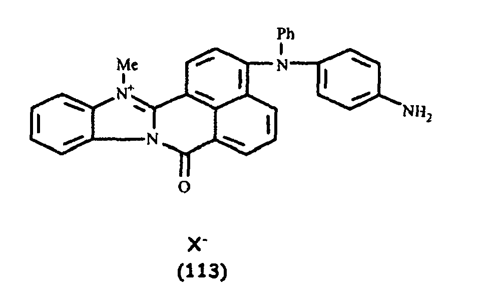

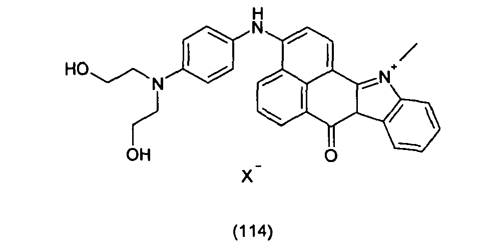

6. Dye composition for dyeing keratin fibres, in particular human keratin fibres such as the hair, comprising, in a suitable dyeing medium, in addition to the direct dye(s) of general formula (I) as defined in one of the preceding claims, at least one oxidation dye precursor and/or at least one conditioning agent and/or at least one thickening polymer and/or at least one surfactant.

7. Dye composition according to Claim 6, such that it comprises from 0.001% to 20%, preferably from 0.1% to 10% and even more preferably from 0.1% to 5% of direct dye(s) of formula (I) relative to the total weight of the composition.

8. Dye composition according to Claim 6 or 7, in which the conditioning agent(s) is (are) chosen from cationic polymers, cations, silicones, chitosans and chitosan derivatives.

9. Dye composition according to one of Claims 6 to 8, in which the surfactant(s) is (are) chosen from the group formed by anionic surfactants, amphoteric or zwitterionic surfactants, nonionic surfactants and cationic surfactants.

10. Dye composition according to one of Claims 6 to 9, in which the oxidation dye precursor(s) is (are) chosen from oxidation bases and couplers.

11. Dye composition according to Claim 10, in which the oxidation base(s) is (are) chosen from para-phenylenediamines, bis(phenyl)alkylenediamines, para-aminophenols, ortho-aminophenols and heterocyclic bases, and the addition salts thereof.

12. Dye composition according to Claim 10 or 11, in which the oxidation base(s) is (are) present in an amount of between 0.001% and 20% by weight and preferably between 0.005% and 6% by weight relative to the total weight of the composition.

13. Dye composition according to one of Claims 10 to 12, in which the coupler(s) is (are) chosen from meta-phenylenediamines, meta-aminophenols, meta-diphenols, naphthalene-based couplers and heterocyclic couplers, and the addition salts thereof.

14. Composition according to Claim 13, in which the coupler(s) is (are) chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene and 2,6-bis(β-hydroxyethylamino)toluene, and the addition salts thereof.

15. Composition according to Claim 13 or 14, in which the coupler(s) is (are) present in an amount of between 0.001% and 20% and preferably between 0.005% and 6% by weight relative to the total weight of the composition.

16. Composition according to one of the preceding claims, such that it comprises at least one additional direct dye.

17. Composition according to one of the preceding claims, such that it comprises at least one hydroxylated solvent such as ethanol, propylene glycol, glycerol or polyol monoethers.

18. Composition according to one of the preceding claims, such that it comprises at least one oxidizing agent chosen from hydrogen peroxide, urea peroxide, alkali metal bromates, persalts, peracids and oxidase enzymes, and preferably hydrogen peroxide.

19. Process for dyeing keratin fibres, such that it includes the steps of applying the dye composition according to one of Claims 6 to 18 to the keratin fibres, and then leaving it to act for a period of between 5 minutes and 1 hour and preferably between 15 minutes and 1 hour.

20. Use of the composition according to one of Claims 6 to 18 for obtaining on keratin fibres, in particular human keratin fibres such as the hair, very strong, sparingly selective and fast shades.

## Patentansprüche

1. Verwendung einer Färbezusammensetzung zum Färben von Keratinfasern, insbesondere von menschlichen Keratinfasern, wie der Haare, die in einem zum Färben geeigneten Medium mindestens einen Direktfarbstoff der allgemeinen Formel (I) enthält, 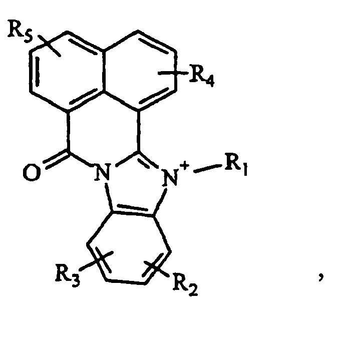 in der bedeuten:
R₁ eine geradkettige oder verzweigte C₁-C₁₀-Alkylgruppe oder eine C₁-C₁₀-Arylalkylgruppe,
R₂ und R₃ unabhängig voneinander ein Wasserstoffatom, eine geradkettige oder verzweigte C₁-C₁₀-Alkylgruppe, eine geradkettige oder verzweigte C₁-C₁₀-Alkoxygruppe, ein Halogenatom (F, Cl, Br, I), eine C₁-C₁₀-Alkoxycarbonylgruppe oder eine Gruppe CF₃,
R₄ und R₅ unabhängig voneinander ein Wasserstoffatom, ein Halogenatom, eine geradkettige oder verzweigte C₁-C₁₀-Alkylgruppe, eine geradkettige oder verzweigte C₁-C₁₀-Alkoxygruppe, eine Hydroxygruppe, eine Aminogruppe, eine geradkettige oder verzweigte C₁-C₁₀-Alkylaminogruppe, eine geradkettige oder verzweigte Di-C₁-C₁₀-alkylaminogruppe, eine Phenylaminogruppe, eine Diphenylaminogruppe, eine C₁-C₄-Alkylaminophenylamino-Gruppe, eine Aminophenylaminophenyl-Gruppe, eine geradkettige oder verzweigte C₁-C₁₀-Alkylthiogruppe, eine Phenylthiogruppe, eine C₁-C₁₀-Alkylphenylthiogruppe, eine C₁-C₁₀-Alkoxyphenylthiogruppe, eine Phenyl-C₁-C₄-alkylthiogruppe oder eine Morpholinogruppe, oder R₄ und R₅ bilden zusammen einen aromatischen oder heterocyclischen Ring mit 5 bis 12 Ringgliedern,
und
X- ein Gegenion einer anorganischen oder organischen Säure.

2. Verwendung der Färbezusammensetzung nach Anspruch 1, wobei in der allgemeinen Formel (I) des enthaltenen Direktfarbstoffs R₁ eine der folgenden Gruppen bedeutet: C₁-C₄-Alkyl; C₁-C₅-Alkyl, das mit einer OH-Gruppe substituiert ist; -CH₂-C₆H₄-CN; -CH₂-C₆H₅.

3. Verwendung der Färbezusammensetzung nach Anspruch 1 oder 2, wobei in der allgemeinen Formel (I) des enthaltenen Direktfarbstoffs R₂ und R₃ unabhängig voneinander bedeuten: ein Wasserstoffatom; Br; Cl; CF₃; C₁-C₄-Alkyl; C₁-C₄-Alkoxy; C₁-C₆-Alkoxycarbonyl; C₁-C₈-Hydroxyalkyl.

4. Verwendung der Färbezusammensetzung nach einem der Ansprüche 1 bis 3, wobei in der allgemeinen Formel I des enthaltenen Direktfarbstoffs R₄ und R₅ unabhängig voneinander bedeuten: ein Wasserstoffatom; eine geradkettige oder verzweigte C₁-C₄-Alkylgruppe; eine C₁-C₄-Alkylaminophenylamino-Gruppe; eine Aminophenylaminophenyl-Gruppe; eine Phenylthiogruppe, eine C₁-C₁₀-Alkylphenylthio-Gruppe; eine C₁-C₁₀-Alkoxyphenylthiogruppe; eine Phenyl-C₁-C₄-alkylthiogruppe oder Morpholino.

5. Verwendung der Färbezusammensetzung nach einem der vorhergehenden Ansprüche, die als Direktfarbstoff mindestens eine Verbindung enthält, die unter den nachstehenden Verbindungen ausgewählt ist, bei denen X- ein Gegenion einer anorganischen oder organischen Säure darstellt:

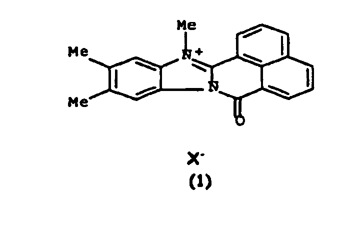

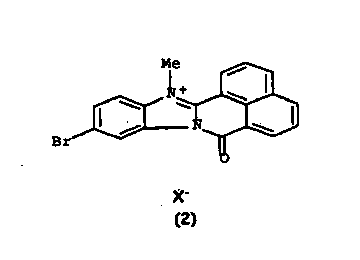

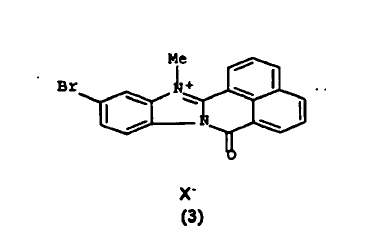

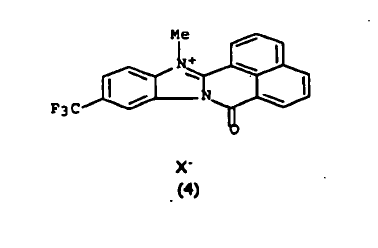

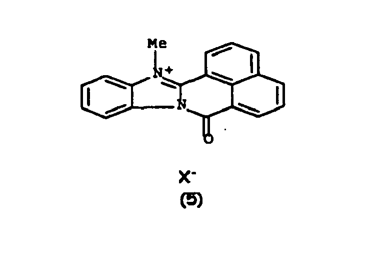

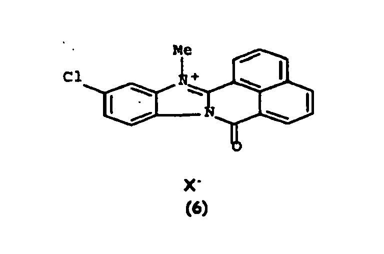

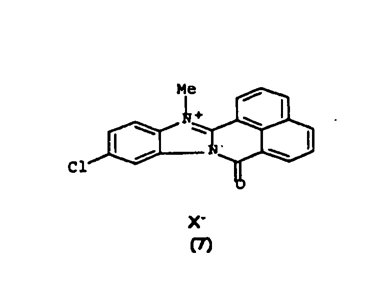

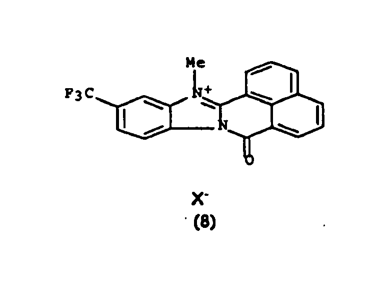

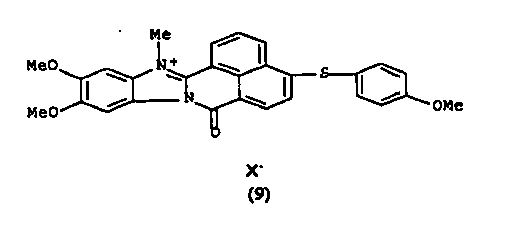

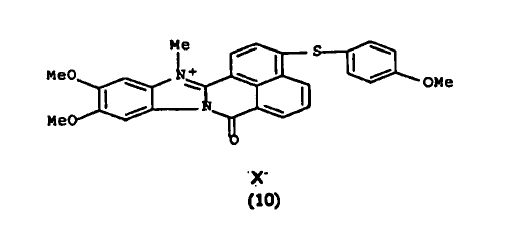

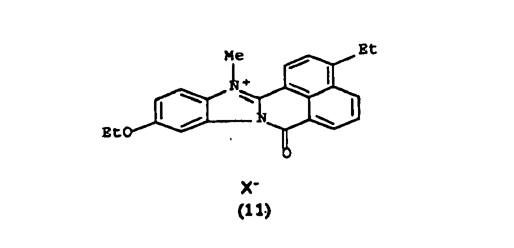

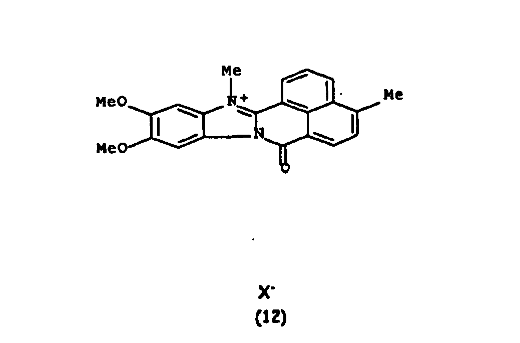

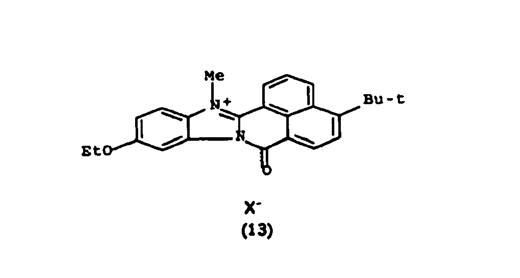

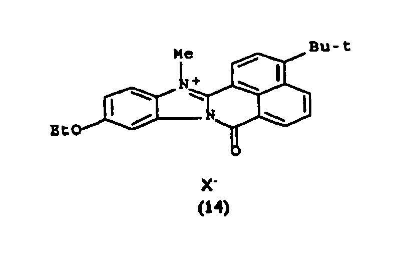

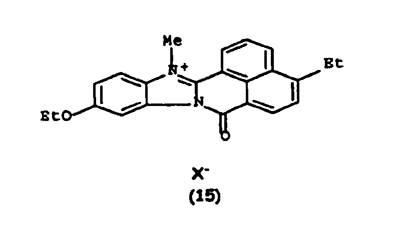

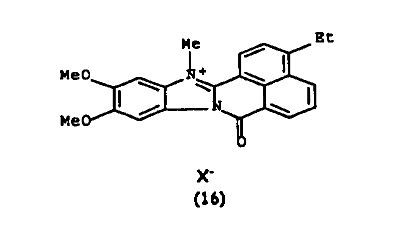

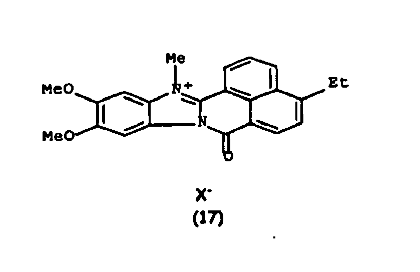

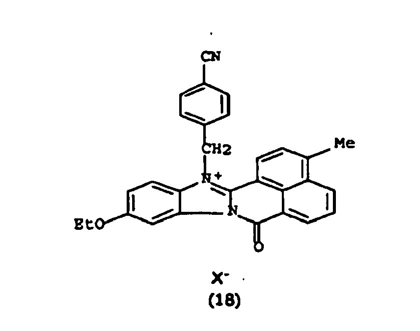

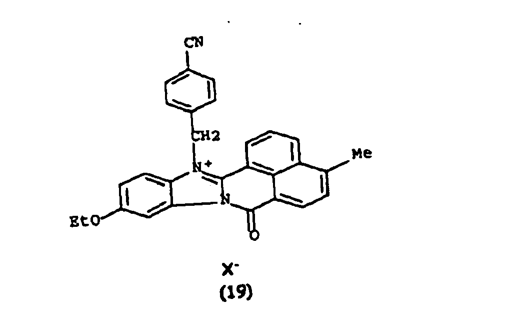

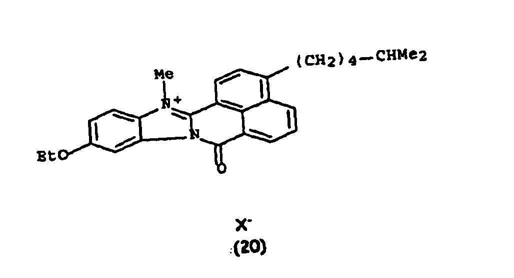

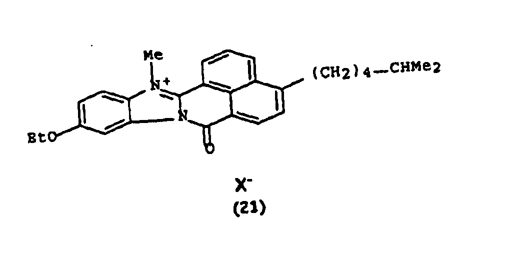

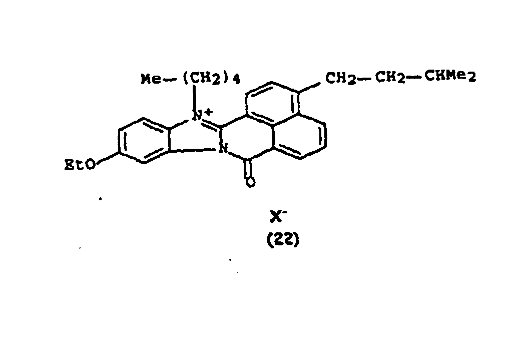

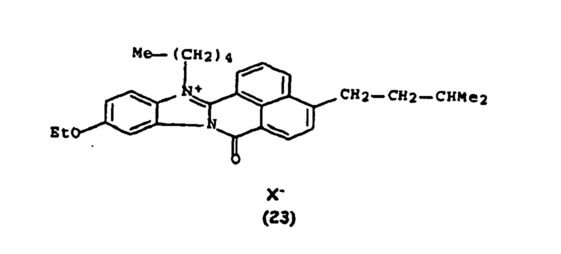

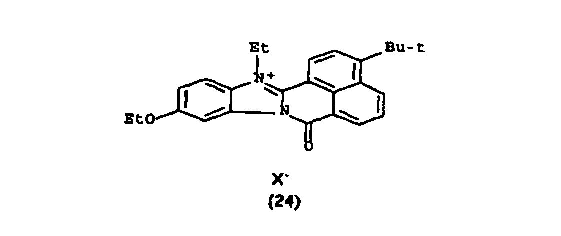

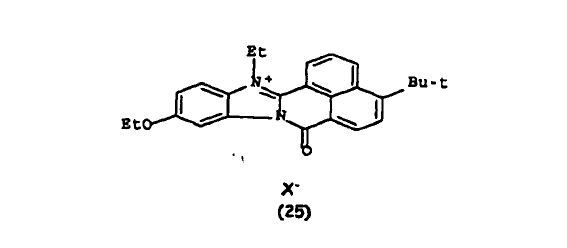

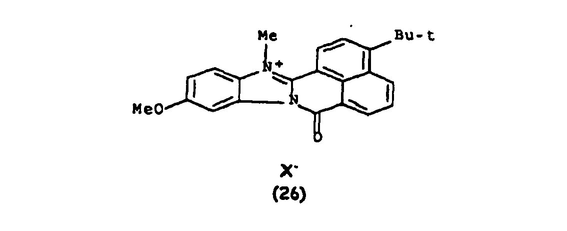

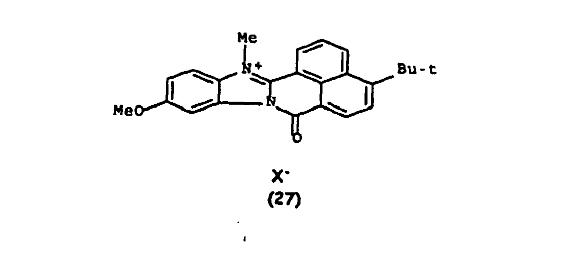

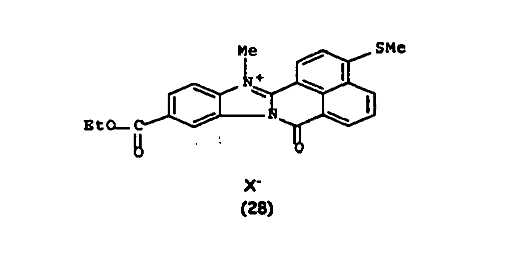

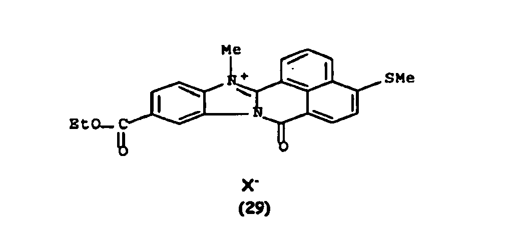

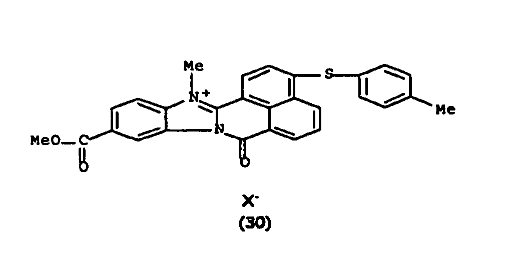

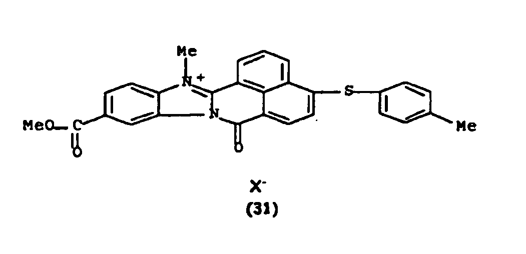

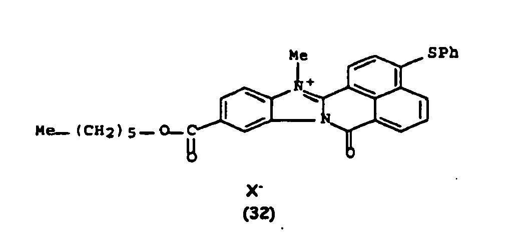

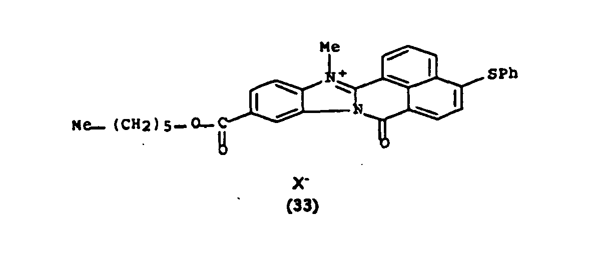

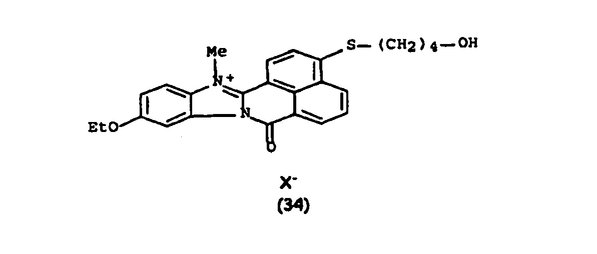

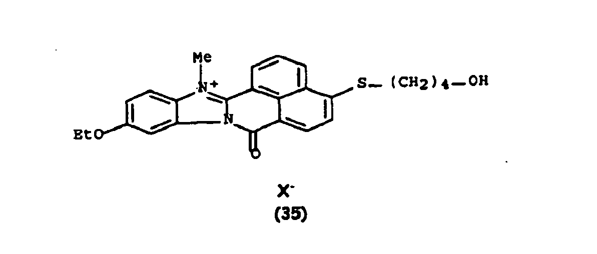

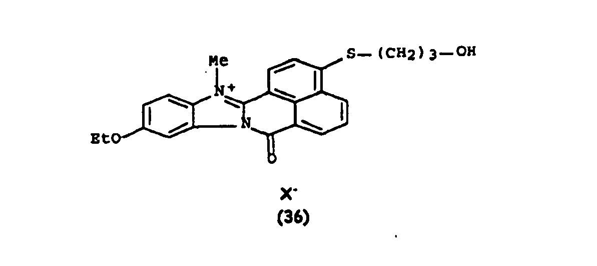

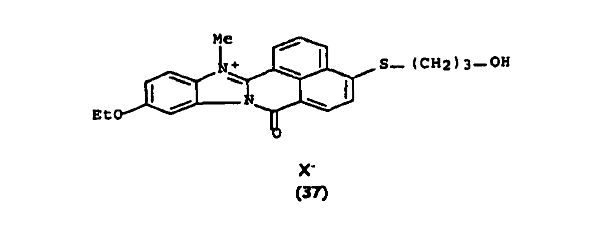

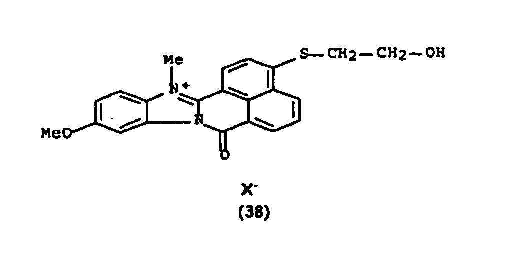

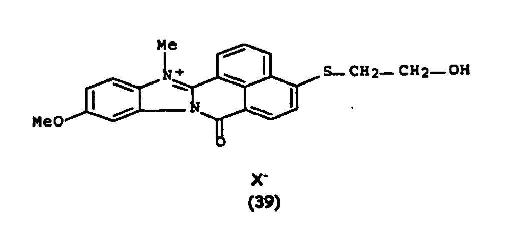

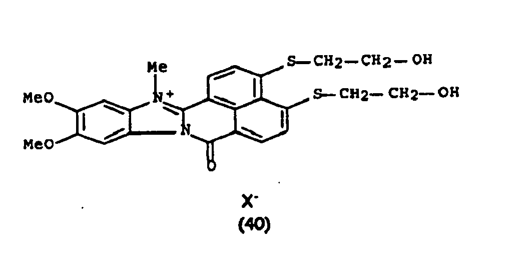

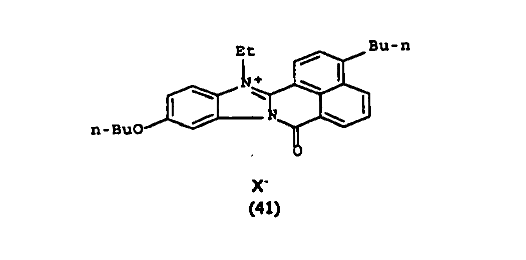

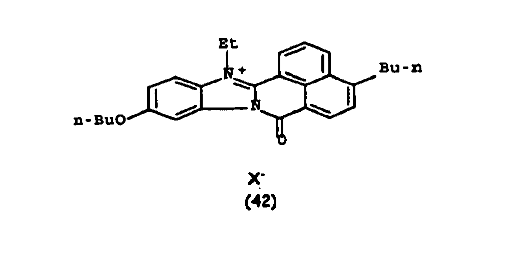

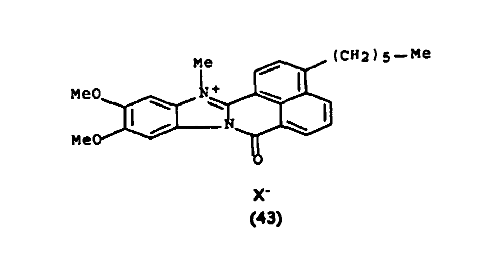

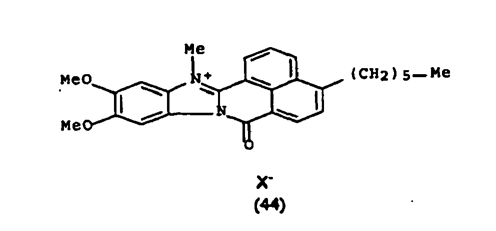

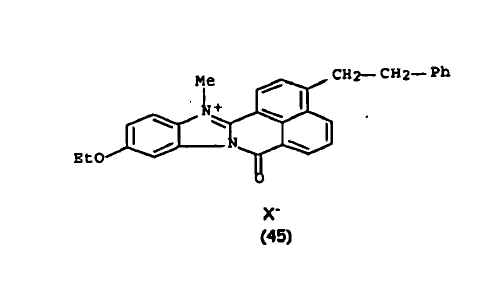

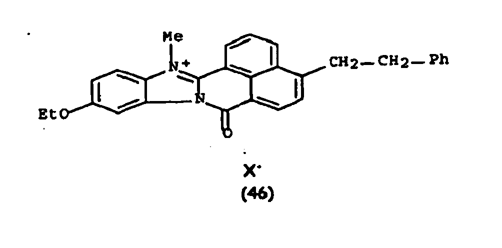

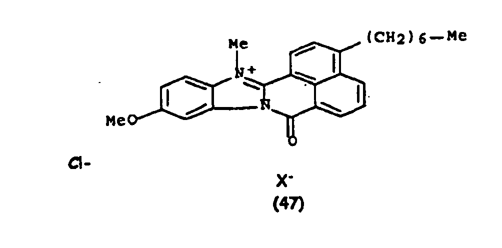

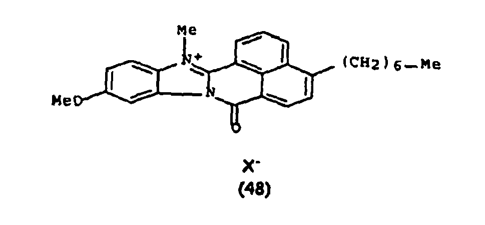

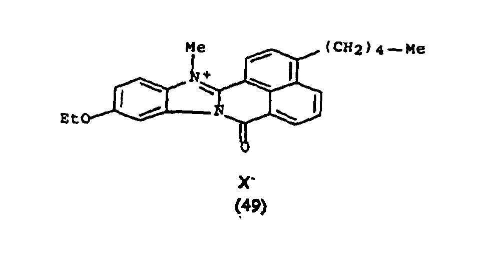

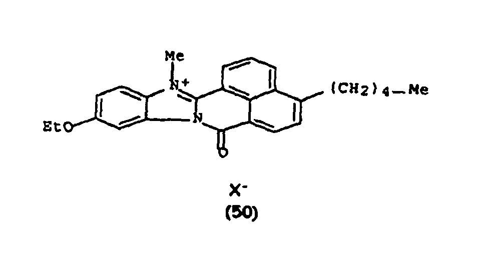

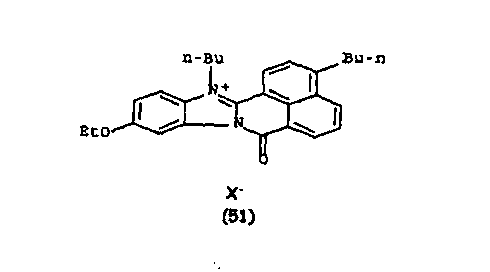

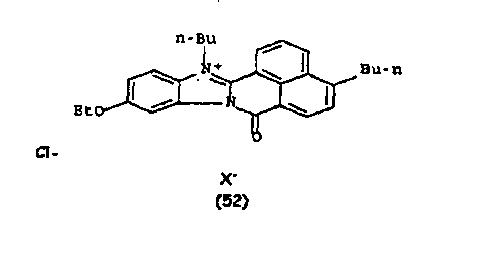

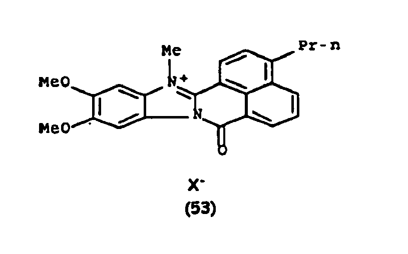

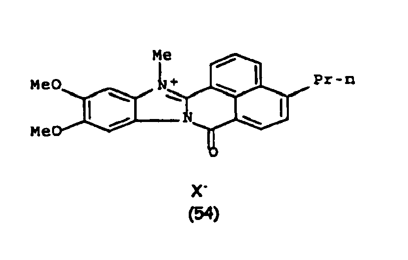

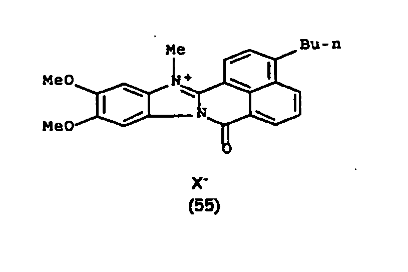

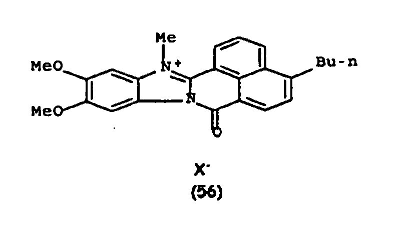

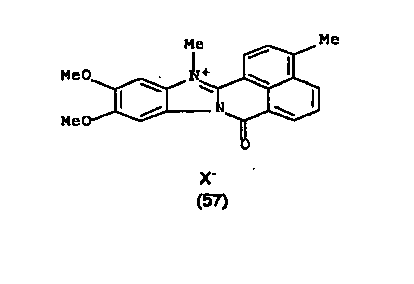

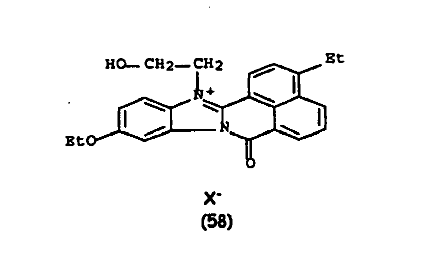

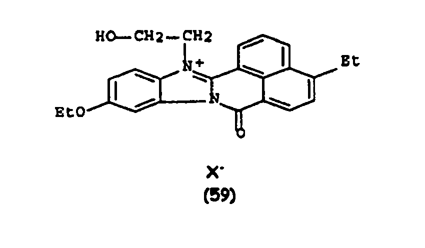

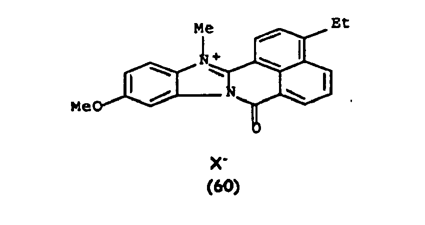

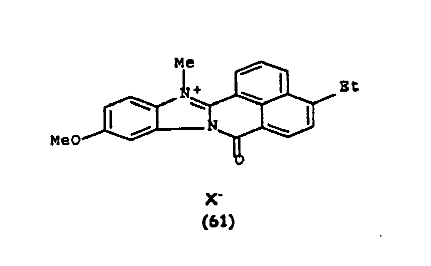

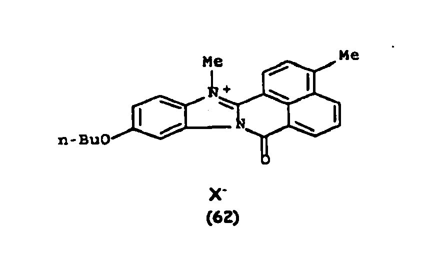

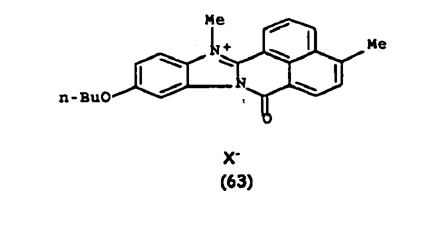

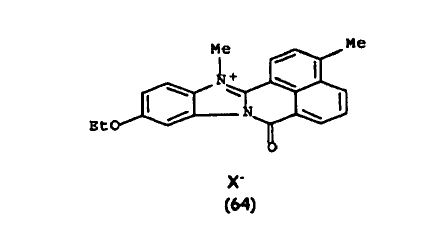

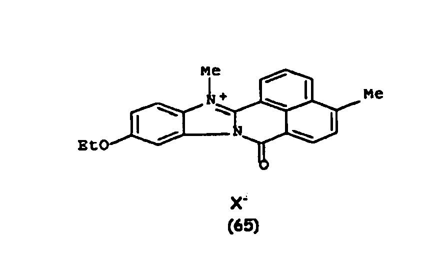

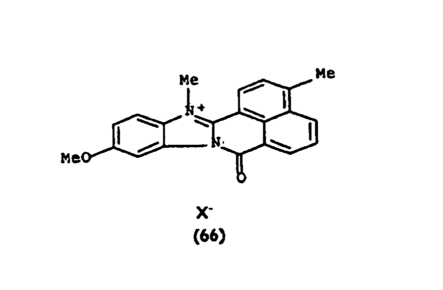

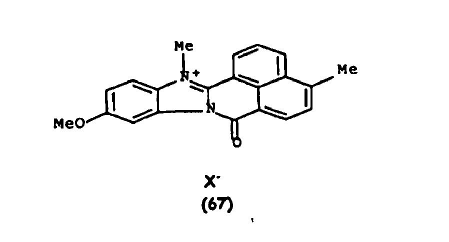

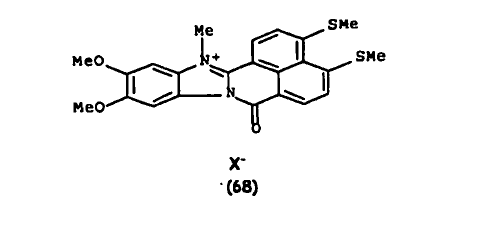

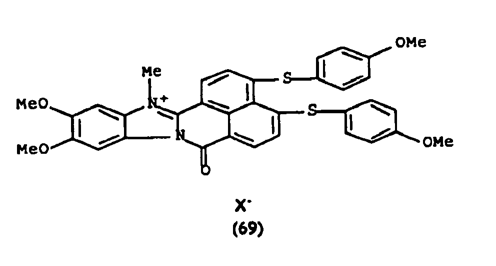

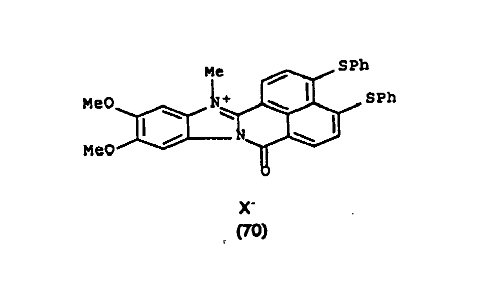

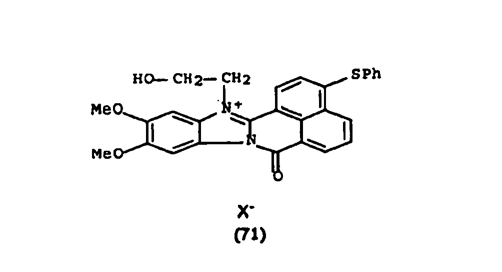

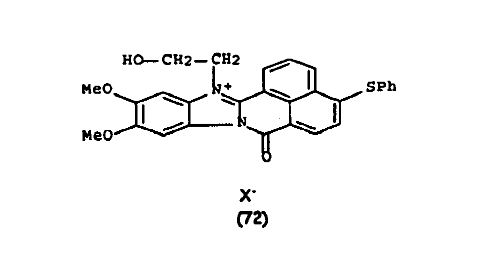

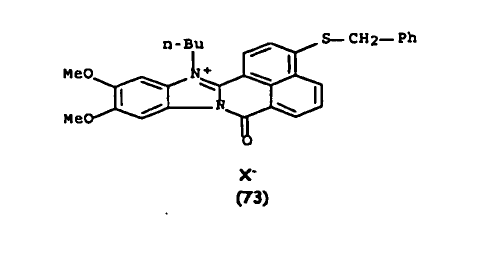

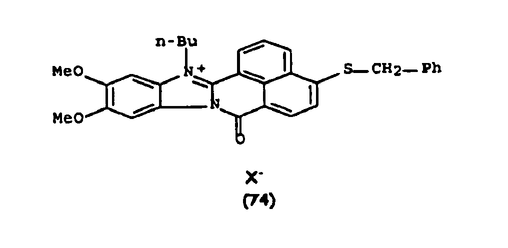

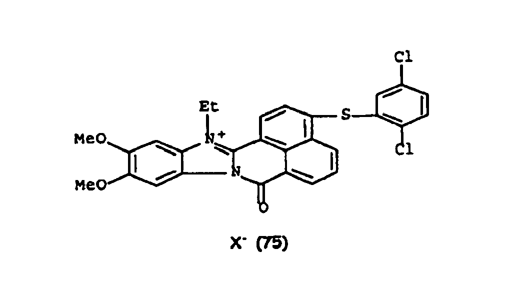

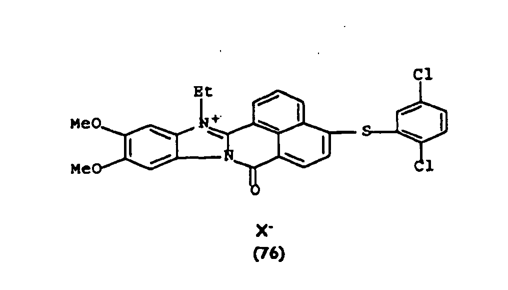

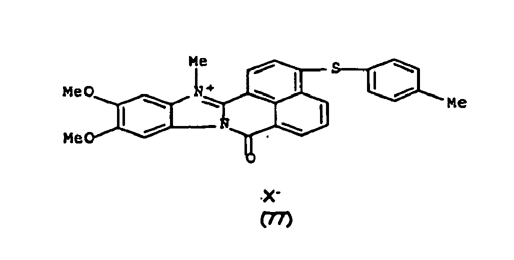

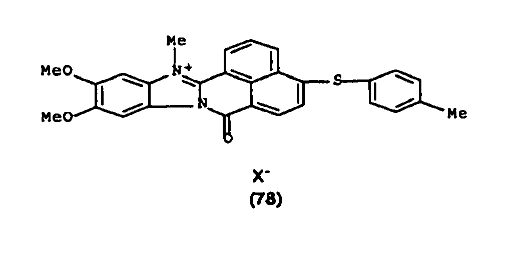

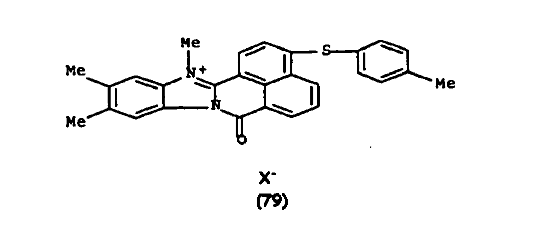

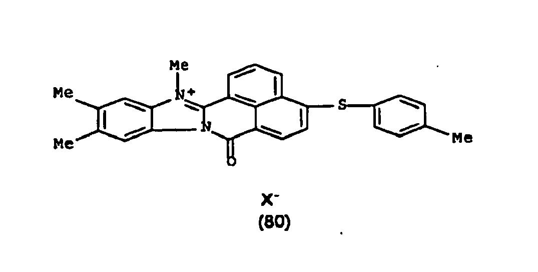

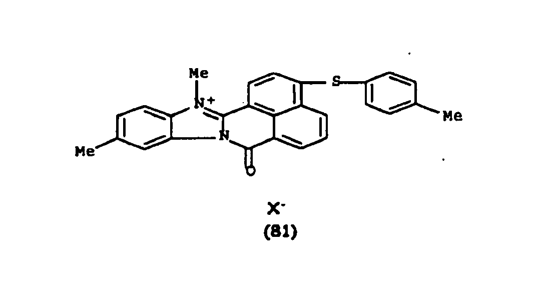

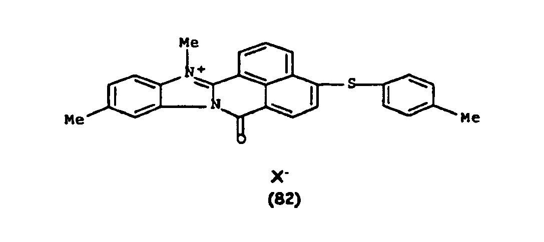

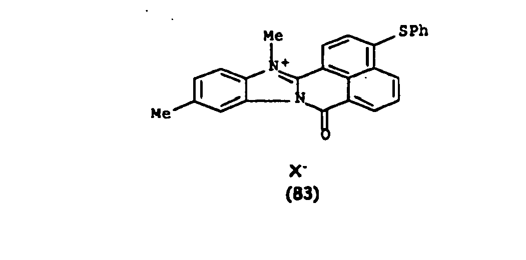

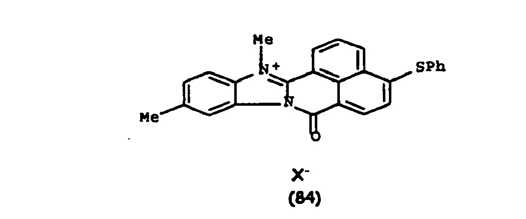

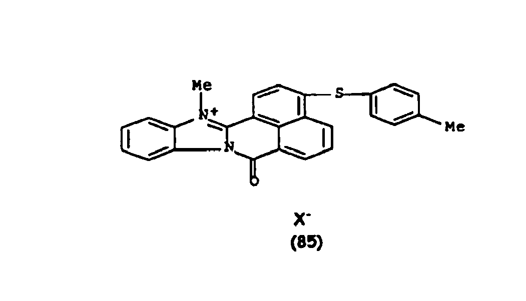

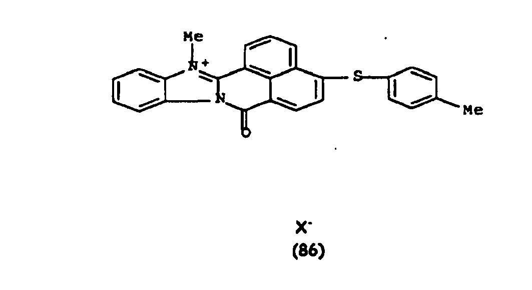

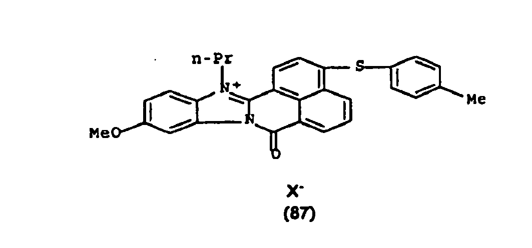

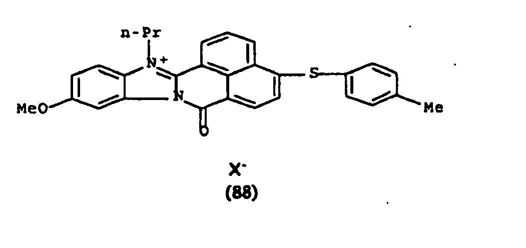

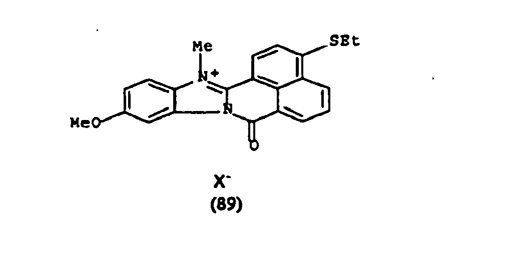

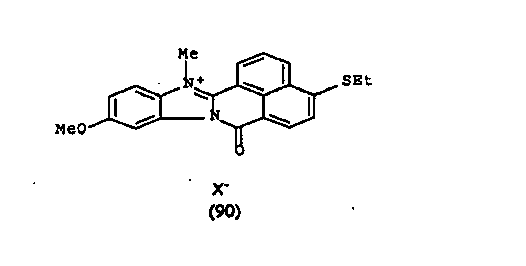

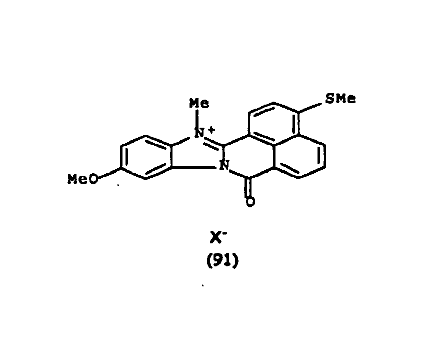

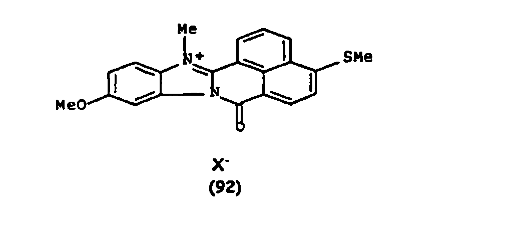

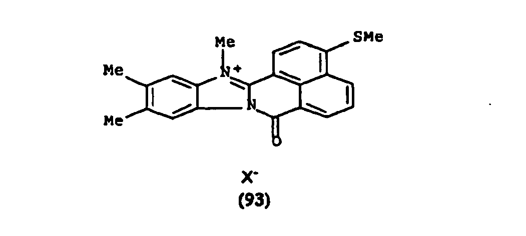

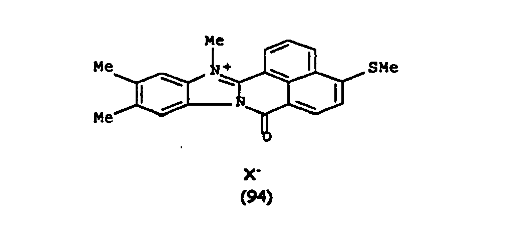

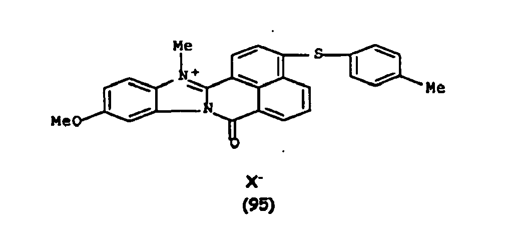

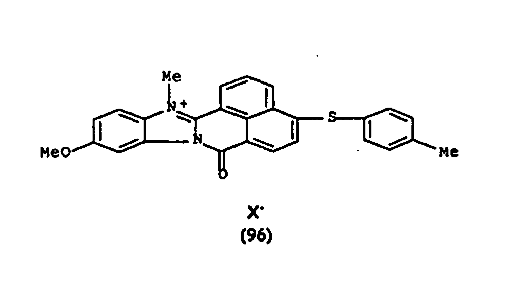

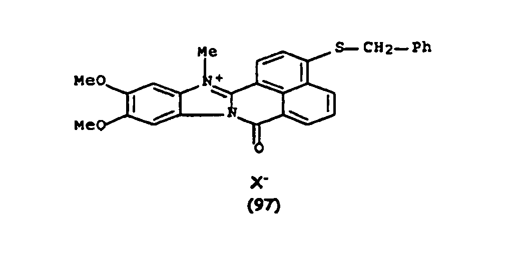

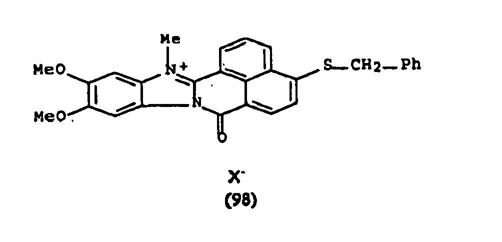

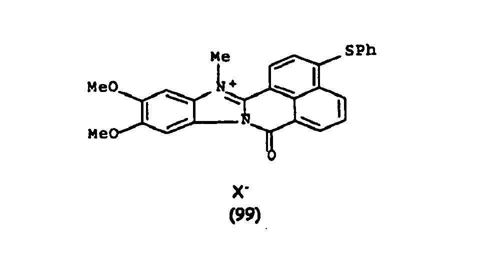

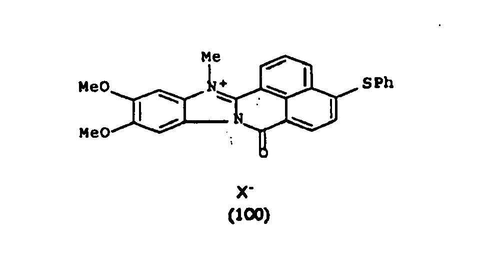

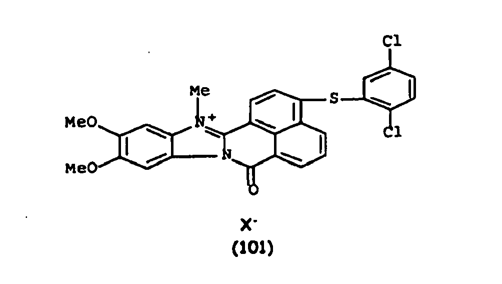

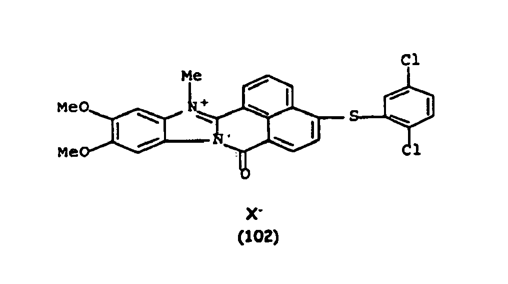

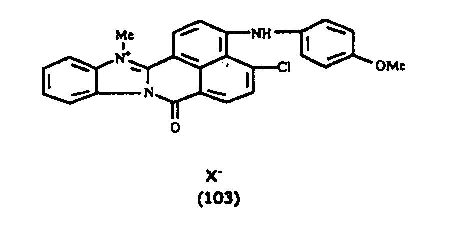

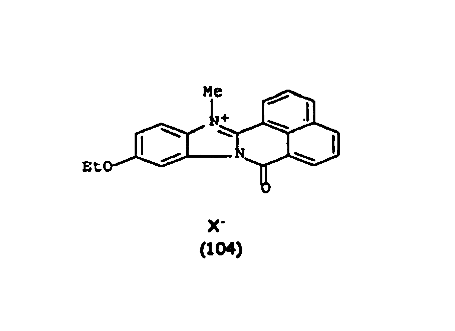

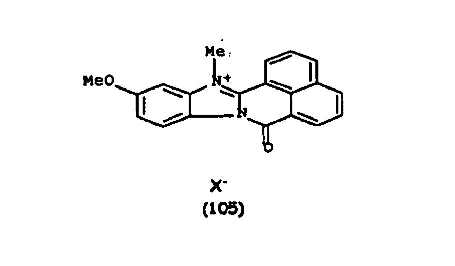

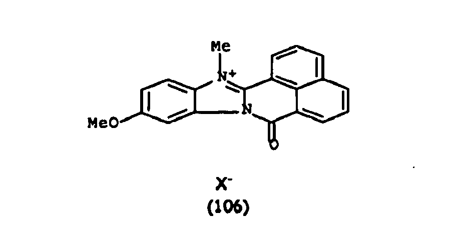

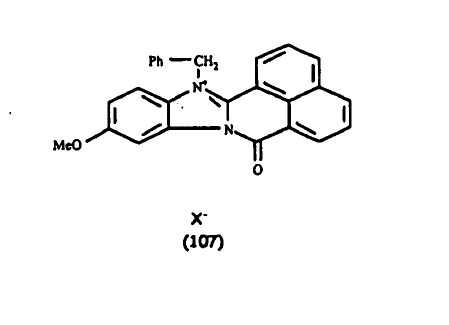

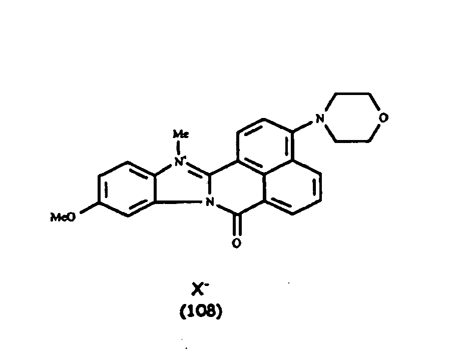

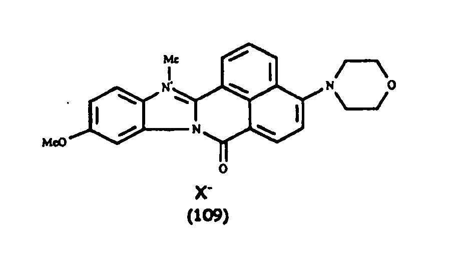

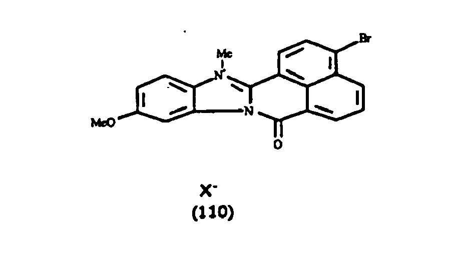

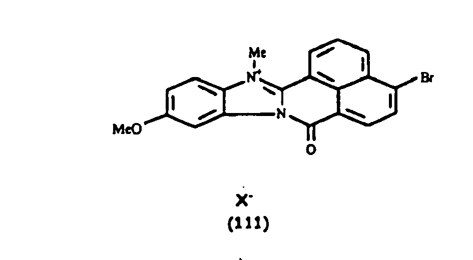

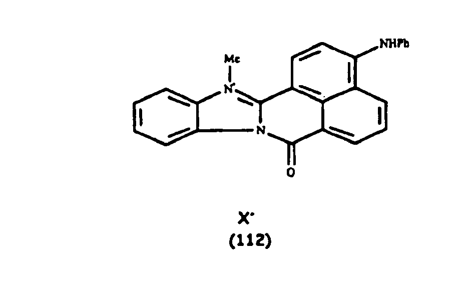

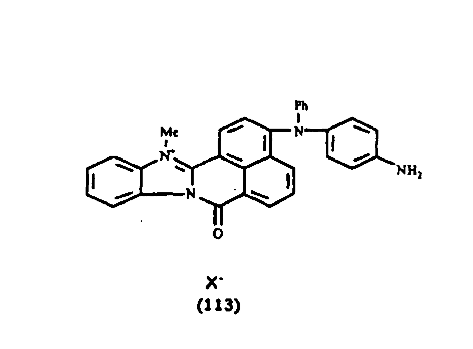

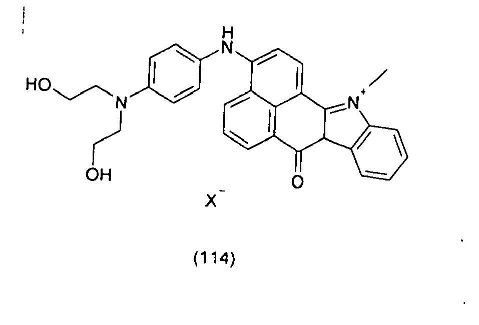

6. Färbezusammensetzung zum Färben von Keratinfasern, insbesondere von menschlichen Keratinfasern wie der Haare, die in einem zum Färben geeigneten Medium neben dem Direktfarbstoff der Formel (I) oder den Direktfarbstoffen der Formel (I), wie sie in einem der vorhergehenden Ansprüche definiert sind, mindestens einen Vorläufer eines Oxidationsfarbstoffs und/oder mindestens ein Konditioniermittel und/oder mindestens ein verdickendes Polymer und/oder mindestens ein Tensid enthält.

7. Färbezusammensetzung nach Anspruch 6, die einen oder mehrere Direktfarbstoffe der Formel (I) in einem Mengenanteil von 0,001 bis 20 %, vorzugsweise in einem Mengenanteil von 0,1 bis 10 % und noch bevorzugter in einem Mengenanteil von 0,1 bis 5 % enthält, bezogen auf das Gesamtgewicht der Zusammensetzung.

8. Färbezusammensetzung nach Anspruch 6 oder 7, bei der das Konditioniermittel oder die Konditioniermittel unter kationischen Polymeren, Kationen, Siliconen, Chitosanen und ChitosanDerivaten ausgewählt sind.

9. Färbezusammensetzung nach einem der Ansprüche 6 bis 8, bei der das Tensid oder die Tenside aus der Gruppe ausgewählt sind, die durch anionische, amphotere, zwitterionische, nichtionische und kationische Tenside gebildet wird.

10. Färbezusammensetzung nach einem der Ansprüche 6 bis 9, bei welcher der oder die Vorläufer eines Oxidationsfarbstoffs unter Oxidationsbasen und Kupplern ausgewählt sind.

11. Färbezusammensetzung nach Anspruch 10, bei der die Oxidationsbase oder die Oxidationsbasen unter p-Phenylendiaminen, Bisphenylalkylendiaminen, p-Aminophenolen, o-Aminophenolen und heterocyclischen Basen sowie ihren Additionssalzen ausgewählt sind.

12. Färbezusammensetzung nach Anspruch 10 oder 11, bei der die Oxidationsbase oder die Oxidationsbasen in einem Mengenanteil von 0,001 bis 20 Gew.-% und bevorzugt in einem Mengenanteil von 0,005 bis 6 Gew.-% vorliegen, bezogen auf das Gesamtgewicht der Zusammensetzung.

13. Färbezusammensetzung nach einem der Ansprüche 10 bis 12, bei welcher der Kuppler oder die Kuppler ausgewählt sind unter m-Phenylendiaminen, m-Aminophenolen, m-Diphenolen, Naphthalinkupplern und heterocyclischen Kupplern sowie ihren Additionssalzen.

14. Zusammensetzung nach Anspruch 13, bei welcher der Kuppler oder die Kuppler ausgewählt sind unter 1,3-Dihydroxybenzol, 1,3-Dihydroxy-2-methylbenzol, 4-Chlor-1,3-dihydroxbenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)-propan, 3-Ureidoanilin, 3-Ureido-1-dimethylaminobenzol, Sesamol, 1-(β-Hydroxyethylamino)-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxypyridin, 6-Hydroxybenzomorpholin, 3,5-Diamino-2,6-dimethoxypyridin, 1-[N-(β-Hydroxyethyl)-amino]-3,4-methylendioxybenzol und 2,6-Bis(β-hydroxy-ethylamino)-toluol sowie den Additionssalzen dieser Verbindungen.

15. Zusammensetzung nach Anspruch 13 oder 14, bei welcher der Kuppler oder die Kuppler in einem Mengenanteil von 0,001 bis 20 Gew.-% und vorzugsweise einem Mengenanteil von 0,005 bis 6 Gew.-% vorliegen, bezogen auf das Gesamtgewicht der Zusammensetzung.

16. Zusammensetzung nach einem der vorhergehenden Ansprüche, die mindestens einen zusätzlichen Direktfarbstoff enthält.

17. Zusammensetzung nach einem der vorhergehenden Ansprüche, die mindestens ein hydroxylgruppenhaltiges Lösungsmittel, wie Ethanol, Propylenglycol, Glycerin und Polyolmonoether, enthält.

18. Zusammensetzung nach einem der vorhergehenden Ansprüche, die mindestens ein Oxidationsmittel enthält, das unter Wasserstoffperoxid, Harnstoffperoxid, Alkalimetallbromaten, Persalzen, Persäuren und Oxidase-Enzymen ausgewählt ist, vorteilhaft Wasserstoffperoxid.

19. Verfahren zum Färben von Keratinfasern, das folgende Stufen umfasst: Aufbringen der Färbezusammensetzung nach einem der Ansprüche 6 bis 18 auf die Keratinfasern und anschließendes Einwirkenlassen während einer Zeitdauer von 5 Minuten bis 1 Stunde und vorzugsweise 15 Minuten bis 1 Stunde.

20. Verwendung der Zusammensetzung nach einem der Ansprüche 6 bis 18 zur Erzielung sehr kräftiger, wenig selektiver und haltbarer Farbnuancen auf Keratinfasern, insbesondere menschlichen Keratinfasern wie den Haaren.
